# EUROPEAN PATENT SPECIFICATION

(11) **EP 2 044 223 B1**
(45) Date of publication and mention of the grant of the patent: **27.06.2012**
(21) Application number: 07836260.5
(22) Date of filing: 26.07.2007
(51) Int. Cl.: C12Q 1/68

(54) **DIAGNOSIS AND TREATMENT OF AGE RELATED MACULAR DEGENERATION**
DIAGNOSE UND BEHANDLUNG ALTERSBEDINGTER MAKULADEGENERATION
DIAGNOSTIC ET TRAITEMENT DE LA DÉGÉNÉRESCENCE MACULAIRE

(30) Priority: 26.07.2006 US 833497 P; 22.03.2007 US 919409 P
(43) Date of publication of application: 08.04.2009
(73) Proprietor: Yale University, Inc., New Haven CT 06511 (US)
(72) Inventor: HOH, Josephine, Westport, CT 06880 (US); DEWAN, Andrew, New York, NY 10009 (US)
(74) Representative: Lock, Graham James
(86) International application number: PCT/US2007/016809
(87) International publication number: WO 2008/013893

(56) References cited:
- WO-A-2006/062716
- WO-A-2006/133295
- DATABASE DBSNP [Online] NCBI; refSNP cluster report 16 March 2004 (2004-03-16), XP002474778 retrieved from HTTP://WWW.NCBI.NLM.NIH.GOV/SNP/SNP_REF.CG I?RS=11200638 Database accession no. rs11200638
- JAKOBSDOTTIR JOHANNA ET AL: "Susceptibility genes for age-related maculopathy on chromosome 10q26" AMERICAN JOURNAL OF HUMAN GENETICS, AMERICAN SOCIETY OF HUMAN GENETICS, CHICAGO, IL, US, vol. 77, no. 3, September 2005 (2005-09), pages 389-407, XP002419863 ISSN: 0002-9297
- HADDAD ET AL: "The Genetics of Age-Related Macular Degeneration: A Review of Progress to Date" SURVEY OF OPHTHALMOLOGY, SURVEY OF OPHTHALMOLOGY INC, XX, vol. 51, no. 4, 3 July 2006 (2006-07-03), pages 316-363, XP005527236 ISSN: 0039-6257
- CHIEN JEREMY ET AL: "A candidate tumor suppressor HtrA1 is downregulated in ovarian cancer." ONCOGENE, vol. 23, no. 8, 26 February 2004 (2004-02-26), pages 1636-1644, XP002474777 ISSN: 0950-9232
- YANG ZHENGLIN ET AL: "A variant of the HTRA1 gene increases susceptibility to age-related macular degeneration" SCIENCE, AMERICAN ASSOCIATION FOR THE ADVANCEMENT OF SCIENCE,, US, vol. 314, no. 5801, 10 November 2006 (2006-11-10), pages 992-993, XP002460077 ISSN: 0036-8075

## Description

### GOVERNMENT FUNDING

This invention was made with Government support from the National Institutes of Health under Grant No. R01EY15771.

### BACKGROUND OF INVENTION

Age related macular degeneration (AMD) is the leading cause of vision loss and blindness among older individuals in the United States and throughout the developed world. It has a complex etiology involving genetic and environmental factors. AMD is broadly classified as either dry (non-neovascular) or wet (neovascular). The dry form is more common, accounting for approximately 85%-90% of patients with AMD, and does not typically result in blindness. The primary clinical sign of dry AMD is the presence of soft drusen with indistinct margins (extracellular protein deposits) between the retinal pigment epithelium (RPE) and Bruch's membrane. The accumulation of these drusen is associated with central geographic atrophy (CGA) and results in blurred central vision. About 10% of AMD patients have the wet form, in which new blood vessels form and break beneath the retina (choroidal neovascularization [CNV]). This leakage causes permanent damage to surrounding retinal tissue, distorting and destroying central vision. Why some individuals develop the more aggressive wet form of AMD, while others have the slowly progressing dry type, is not well understood.

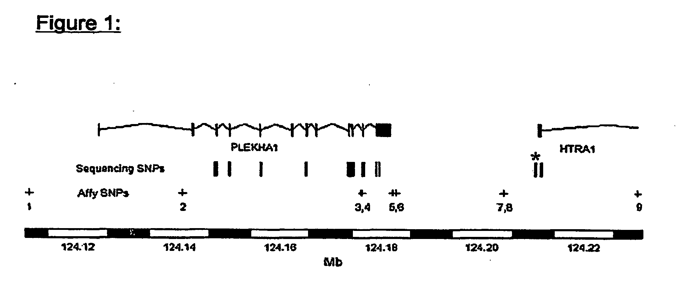

Jakobsdottir et al., 2005 (American Journal of Human Genetics 77(3): 389-407) discloses a method of identifying an individual at risk of developing age-related macular degeneration (AMD) comprising assaying the SNP defined by rs10490924 in the theoretical gene LOC387715, adjacent to the regulatory region of HTRA1 (see Materials and Table 6). The genotyping is done using allele-specific probes in a high-density SNP arrays (page 392, col. 2). This document also studies several SNPs in the PRSS11 gene, an alternative name for HTRA1, for association to AMS (see Figure 1 and Table 6).

### SUMMARY OF THE INVENTION

The present invention relates to identification of a variation in a human gene correlated with the occurrence of age related macular degeneration; which is useful in identifying or aiding in identifying individuals at risk for developing age related macular degeneration, as well as for diagnosing or aiding in the diagnosis of age related macular degeneration (identifying or aiding in identifying individuals suffering from age related macular degeneration). The methods and compositions are also useful to monitor the status (e.g., progression or reversal) of age related macular degeneration. The methods and compositions of the present invention are useful to identify or aid in identifying individuals of a variety of races and ethnicities and, in particular embodiments, are carried out in order to identify or aid in identifying Caucasian or Asian individuals suffering from or at risk of developing age related macular degeneration. The invention also relates to methods for identifying or aiding in identifying individuals suffering from or at risk for developing age related macular degeneration and methods for diagnosing or aiding in the diagnosis of age related macular degeneration (identifying individuals suffering from/individuals who have age related macular degenerations).

The present invention provides methods, diagnostic kits and compositions as defined in the claims.

The methods of this invention can comprise, in addition to determining whether an HTRA1 variant is present, determining whether one or more additional variants that are correlated with the occurrence of age related macular degeneration is present in an individual being assessed. Additional variants, other than an HTRA1 variant, that can be detected include, but are not limited to, a variation in nucleic acids (DNA, RNA) encoding the CFH protein (e.g., a variation encoding histidine at position 402 of the CFH protein); a variation encoding an amino acid residue other than alanine at position 69 of the protein LOC387715 (e.g., a serine at that position); and a variation corresponding to the single nucleotide polymorphism identified as rs10490924. Some or all of these variants, as well as others correlated with the occurrence of age related macular degeneration, can be determined and/or quantified in a sample from an individual being assessed.

The invention relates to a method of monitoring the status of age related macular degeneration in an individual (human). The method is useful to assess, for example, whether age related macular degeneration has progressed (reached a more advanced or later stage) in an individual. This is useful, for example, in assessing the effects/effectiveness of treatments an individual has received. The methods of this invention can help show, for example, that a treatment has been effective, in that it can show if regression (amelioration, partial or complete) of AMD has occurred. The method can also be used to assess whether AMD in an individual has progressed (worsened). This embodiment can be carried out, for example, by assessing the extent to which a variant HTRA1 gene comprising a variation in a noncoding region as described herein is present in a sample obtained from the individual. If the variant HTRA1 is present in the sample to a lesser extent following treatment (than prior to treatment), this is an indication of regression of AMD and that treatment was effective.

### BRIEF DESCRIPTION OF THE DRAWINGS

Figure 1 is a schematic depicting the genes in the 4-gamete region on chromosome 10q26, as well as the location of SNPs genotyped by microarrays (+) and identified through sequencing (|). SNP rs10490924 is labeled as "8" and rs11200638 is marked with an asterisk.

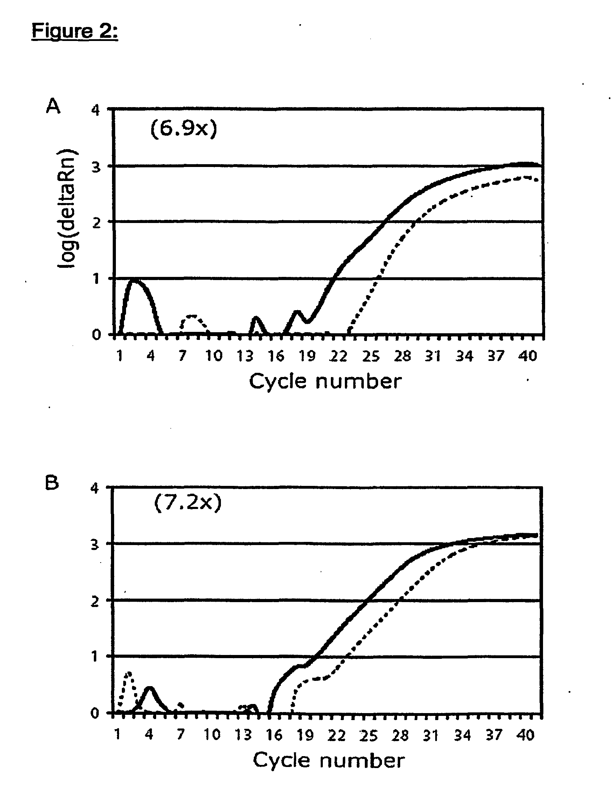

Figures 2A-B are graphs depicting results obtained by quantitative PCR (qPCR) of ChIP DNA prepared from HeLaS3 cells: Figure 2A AP-2α (solid line), Figure 2B SRF (solid line), and (Figure 2A and 2B) normal rabbit immunoglobulin G (dashed line) represent the immunoprecipitations analyzed. Positive and negative control promoters were also tested. The log(ΔRn) (y-axis) is plotted against the PCR cycle number (x-axis). The ΔΔCt values fold increase of transcription calculated relative to reference PCR reactions) are shown in parentheses.

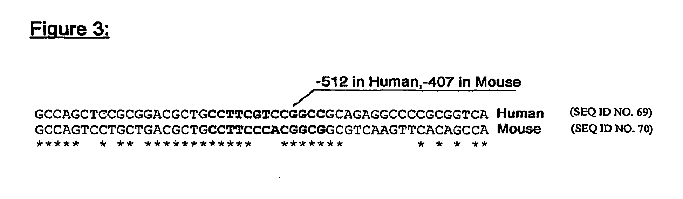

Figure 3 is a depiction of a computation analysis of the HTRA1 promoter sequence. Human and Mouse orthologous sequences in the HTRA1 promoter; the conserved nucleotides are marked with *.

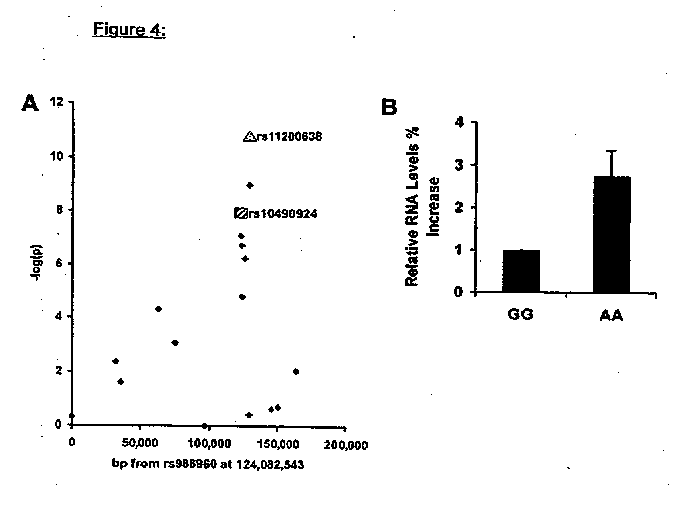

Figure 4A is a plot depicting log P-values (y axis) from association analyses for the 15 SNPs at the 10q AMD region using 442 AMD cases and 309 controls (see also table 2). For the rs10490924 (square) and rs11200638 (triangle) SNPs, associations were derived from a larger sample size (581 AMD cases).
Figure 4B is a bar graph depicting results obtained by Real-time RT-PCR semi-quantitative analysis of HTRA1 RNA levels in blood lymphocytes from three AMD patients with the AA genotype and three normal controls with the GG genotype. The statistical significance of the differences in expression level was examined using an independent samples t test (SPSS version 13.0): AA:GG (P =0.02). The error bars indicate the 95.0% confidence interval of the mean.

### DETAILED DESCRIPTION OF THE INVENTION

The discovery that a variation in the non-coding region of the HTRA1 gene is associated with AMD is useful for the diagnosis and treatment of individuals, such as those suffering from or at risk of developing age related macular degeneration. The determination of the genetic constitution of the HTRA1 gene in an individual is useful as the basis for diagnosing or treating AMD at earlier stages, or even before an individual displays symptoms of AMD. Furthermore, diagnostic tests to genotype HTRA1 may allow individuals to alter their behavior to minimize environmental risks to AMD (e.g., smoking, obesity). The present invention relates to the identification of a variant HTRA1 gene correlated with the occurrence of AMD, which is useful in (identifying or aiding in identifying individuals at risk for developing AMD, as well as for diagnosing or aiding in the diagnosis of AMD. It also relates to methods for identifying or aiding in identifying individuals at risk for developing AMD, methods for diagnosing or aiding in the diagnosis of AMD, methods for monitoring the status (e.g., progression, reversal) of AMD, polynucleotides (e.g., probes, primers) useful in the methods, diagnostic kits containing probes or primers, methods of treating an individual at risk for or suffering from AMD and compositions useful for treating an individual at risk for or suffering from AMD.

Applicants have shown that a common variation in the non-coding region of the human HTRA1 gene is strongly associated with AMD. The present invention relates to methods and compositions for detecting such variations that are correlated with the occurrence of age related macular degeneration in humans.

HtrA (high temperature requirement) was initially identified in *E. coli* as a heat shock protein and was subsequently found to exist ubiquitously in microbes, plants and animals. Human HTRA1 is a member of the HtrA family of serine proteases. Its structural features include a highly conserved trypsin-like serine protease domain, as well as an insulin-like growth factor binding protein domain and a Kazal-type serine protease inhibitor motif.

Down regulation of human HTRA1 gene expression has been observed in certain cancers (ovarian cancer, melanoma), in close correlation with malignant progression and metastasis of these tumors. Overexpression of HTRA1 in tumors on the other hand suppresses proliferation and migration of tumor cells, suggesting that HTRA1 has tumor suppressive properties in certain cancers. In contrast to tumor tissue, HTRA1 expression is upregulated in skeletal muscle of Duchenne muscular dystrophy and in cartilage of osteoarthitic joints, which may contribute to the development of this disease.

The variation in the non-coding region of the human HTRA1 gene that is strongly associated with AMD described herein is the variation that corresponds to the single nucleotide polymorphism identified as rs 11200638. The variation is a single nucleotide polymorphism (G→A) in the promoter region of the human HTRA1 gene. A single nucleotide polymorphism located in the non-coding regulating region at position -512 relative to the putative transcription start site of the human HTRA1 gene on human chromosome 10 is associated with the risk of developing age related macular degeneration (AMD). This single nucleotide polymorphism (SNP) is identified as rs 11240638. Based on this association as disclosed herein, it is possible to determine whether an individual is at risk of developing AMD using diagnostic tests that can be conducted routinely and reproducibly on a variety of samples from the individual. If the HTRA1 variant is detected in a sample using a diagnostic test, this finding can be used to determine whether an individual is at risk of developing AMD, aid in diagnosing AMD or confirm an AMD diagnosis based on other data.

The promoter region is the regulatory region of a gene that is a non-coding coding section that is not translated into a protein sequence. Certain cellular transcription factors can bind to the promoter region of a gene to influence its transcriptional activity. The single nucleotide polymorphism that is identified as rs11200638 is located at position -512 relative to the putative transcription start site.

Polymorphisms in the promoter region can, under certain circumstances, alter the ability of transcription factors to bind to the promoter, for example by changing the affinity of a transcription factor binding site located within the promoter sequence, for the corresponding transcription factor.

Changes in transcription factor binding to the promoter can affect the activity of a promoter, for example transcriptional activity of the promoter, which can influence the rate of transcription of a gene. A higher rate of transcription can lead to more corresponding protein to be made. A lower rate of transcription can lead to less corresponding protein being made. Changes in protein levels can affect many biological processes and can potentially have debilitating effects.

The term "G→A", as used herein, means a change of a single nucleotide base from a wildtype G to a variant A at a certain position within the genome. Such changes are known in the art as "single nucleotide polymorpisms" or SNP(s). Such changes can affect one or both alleles and this can occur in a heterozygoes or homozygoes manner. The "G→A" change, as used herein, is understood to include both possible genotypes; a heterozygoes G→A change and a homozygoes GG→AA change, even though it is herein referred to only as G→A for reasons of simplicity.

An HTRA1 gene can be the cDNA or the genomic form of the gene, which may include upstream and downstream regulatory sequences, such as promoter sequences. The HTRA1 polypeptide can be encoded by a full length coding sequence or by any portion of the coding sequence, so long as the desired activity or functional properties (e.g., enzymatic activity, ligand binding, signal transduction, etc.) of the full-length or fragment are retained. The HTRA1 gene may further include sequences located adjacent to the coding region on both the 5' and 3' ends for a distance of about 1-2 kb on either end such that the gene corresponds to the length of the full-length mRNA. The sequences which are located 5' of the coding region and which are present on the mRNA are referred to as 5' non-translated sequences. The sequences which are located 3' or downstream of the coding region and which are present on the mRNA are referred to as 3' non-translated sequences.

To provide an overall understanding of the invention, certain illustrative embodiments will now be described, including compositions and methods for identifying or aiding in identifying individuals at risk for developing AMD, as well as for diagnosing or aiding in the diagnosis of AMD. However, it will be understood by one of ordinary skill in the art that the compositions and methods described herein may be adapted and modified as is appropriate for the application being addressed and that the compositions and methods described herein may be employed in other suitable applications, and that such other additions and modifications will not depart from the scope hereof.

### HTRA1 polynucleotide probes and primers

Described herein are isolated and/or recombinant polynucleotides that specifically detect a variation in the non-coding region of the HTRA1 gene that is correlated with the occurrence of AMD. Such as a variation in the HTRA1 promoter is the single nucleotide polymorphism that is identified as rs 11200638. Polynucleotide probes may hybridize to such a variation (referred to as a variation of interest) in a HTRA1 gene in a specific manner and typically have a sequence which is fully or partially complementary to the sequence of the variation. Polynucleotide probes can also hybridize to sequences on either or both sides of the variation of interest; they can hybridize to flanking sequences on either or both sides of the variation of interest. Polynucleotide probes can hybridize to a segment of target DNA such that the variation aligns with a central position of the probe, or the variation may align with another position, such as a terminal position, of the probe.

A polynucleotide probe can hybridize, under stringent conditions, to a nucleic acid molecule comprising a variant HTRA1 gene, or a portion or allelic variant thereof, that is correlated with the occurrence of AMD in humans. For example, a polynucleotide probe hybridizes to a variation in the HTRA1 promoter that is correlated with the occurrence of AMD in humans in a specific example the variation is a nucleotide base other than G at position -512 relative to the putative transcription sart site of the human HTRA1 gene. A polynucleotide probe can hybridize, under stringent conditions, to a nucleic acid molecule (e.g., DNA) of a HTRA1 gene, or an allelic variant thereof, wherein the nucleic acid molecule comprises a variation that is correlated with the occurrence of AMD in humans, such as the variations that is identified as SNP rs 11200638.

A polynucleotide probe may be an allele-specific probe. The design and use of allele-specific probes for analyzing polymorphisms is described by, e.g., Saiki et al., Nature 324:163-166 (1986); Dattagupta, EP 235726; and Saiki WO 89/11548. Allele-specific probes can be designed to hybridize to a segment of a target DNA from one individual and not to hybridize to the corresponding segment from another individual due to the presence of .different polymorphic forms or variations in the respective segments from the two individuals. Hybridization conditions should be sufficiently stringent that there is a significant difference in hybridization intensity between alleles. In some embodiments, a probe hybridizes to only one of the alleles.

A variety of variations in the HTRA1 gene that predispose an individual to AMD can be detected by the methods and polynucleotides described herein. In a specific embodiment the variation in the HTRA1 gene that is correlated with the occurrence of AMD is a variation in the non-coding region of the HTRA1 gene. More specifically the variation is a single nucleotide polymorphism (G→A) at position -512 from the putative transcription start site of the promoter of the human HTRA1 gene. This polymorphism is identified as rs11200638. Polymorphisms other than that at position -512, described above, can be detected in the non-coding region of the human HTRA1 gene particularly within the promoter sequence using the methods and polynucleotides described herein.

Any nucleotide polymorphism of a coding region, exon, exon-intron boundary, signal peptide, 5-prime untranslated region, promoter region, enhancer sequence, 3-prime untranslated region or intron that is associated with AMD can be detected. These polymorphisms include, but are not limited to, changes that: alter the amino acid sequence of the proteins encoded by the HTRA1 gene, produce alternative splice products, create truncated products, introduce a premature stop codon, introduce a cryptic exon, alter the degree or expression to a greater or lesser extent, alter tissue specificity of HTRA1 expression, introduce changes in the tertiary structure of the proteins encoded by HTRA1, introduce changes in the binding affinity or specificity of the proteins expressed by HTRA1 or alter the function of the proteins encoded by HTRA1.

Described herein are polynucleotides that are variants of the polynucleotides described herein, provided that the variant polynucleotides maintain their ability to specifically detect a variation in the non-coding region of the HTRA1 gene, such as a variation in the HTRA1 promoter (e.g., a variation that encodes a change of position -512 from the putative transcription start site or the human HTRA1 gene) that is correlated with the occurrence of AMD. Variant polynucleotides may include, for example, sequences that differ by one or more nucleotide substitutions, additions or deletions.

The isolated polynucleotide may be a probe that hybridizes, under stringent conditions, to a variation in the non-coding region of the HTRA1 gene that is correlated with the occurrence of AMD in humans. The probe may hybridize to a variation that is the single nucleotide polymorphism (G→A) at position -512 from the putative transcription start site of the promoter of the human HTRA1 gene, which is identified as rs11200638. As used herein, the term "hybridization" is used in reference to the pairing of complementary nucleic acids. The term "specifically detects" as used in reference to a polynucleotide is intended to mean, as is generally understood in the art, that the polynucleotide is selective between a nucleic acid of interest and other nucleic acids not of interest. Such a polynucleotide can distinguish between the sequence of a nucleic acid of interest and the sequence of a nucleic acid that is not interest such that the polynucleotide is useful for, at minimum, detecting the presence of the nucleic acid sequence of interest in a particular type of biological sample. The term "probe" refers to a polynucleotide that is capable of hybridizing to a nucleic acid of interest. The polynucleotide may be naturally occurring, as in a purified restriction digest, or it may be produced synthetically, recombinantly or by nucleic acid amplification (e.g., PCR Amplification).

It is well known in the art how to perform hybridization experiments with nucleic acid molecules. The skilled artisan is familiar with the hybridization conditions required in the present invention and understands readily that appropriate stringency conditions which promote DNA hybridization can be varied. Such hybridization conditions are referred to in standard text books, such as Molecular Cloning: A Laboratory Manual, Cold Spring Harbor Laboratory (2001); and Current Protocols in Molecular Biology, eds. Ausubel et al., John Wiley & Sons (1992). Particularly useful in methods of the present invention are polynucleotides which are capable of hybridizing to a variant HTRA1 gene, or a region of a variant HTRA1 gene, under stringent conditions. Under stringent conditions, a polynucleotide that hybridizes to a variant HTRA1 gene does not hybridize to a wildtype HTRA1 gene.

Nucleic acid hybridization is affected by such conditions as salt concentration, temperature, organic solvents, base composition, length of the complementary strands, and the number of nucleotide base mismatches between the hybridizing nucleic acids, as will readily be appreciated by those skilled in the art. Stringent temperature conditions will generally include temperatures in excess of 30°C, or may be in excess of 37°C or 45°C. Stringency increases with temperature. For example, temperatures greater than 45°C are highly stringent conditions. Stringent salt conditions will ordinarily be less than 1000 mM, or may be less than 500 mM or 200 mM. For example, one could perform the hybridization at 6.0x sodium chloride/sodium citrate (SSC) at about 45 °C, followed by a wash of 2.0x SSC at 50 °C. For example, the salt concentration in the wash step can be selected from a low stringency of about 2.0x SSC at 50 °C to a high stringency of about 0.2x SSC at 50 °C. In addition, the temperature in the wash step can be increased from low stringency conditions at room temperature, about 22 °C, to high stringency conditions at about 65 °C. Both temperature and salt may be varied, or temperature or salt concentration may be held constant while the other variable is changed. Particularly useful in methods of the present invention are polynucleotides which are capable of hybridizing to a variant HTRA1 gene, or a region of a variant HTRA1 gene, under stringent conditions. It is understood, however, that the appropriate stringency conditions may be varied in the present invention to promote DNA hybridization. In certain embodiments, polynucleotides of the present invention hybridize to a variant HTRA1 gene, or a region of a variant HTRA1 gene, under highly stringent conditions. Under stringent conditions, a polynucleotide that hybridizes to a variation in the non-coding region of the HTRA1 gene does not hybridize to a wildtype HTRA1 gene. In one embodiment, the invention provides nucleic acids which hybridize under low stringency conditions of 6.0x SSC at room temperature followed by a wash at 2.0x SSC at room temperature. The combination of parameters, however, is much more important than the measure of any single parameter. See, e.g., Wetmur and Davidson, 1968. Probe sequences may also hybridize specifically to duplex DNA under certain conditions to form triplex or higher order DNA complexes. The preparation of such probes and suitable hybridization conditions are well known in the art. One method for obtaining DNA encoding the biosynthetic constructs disclosed herein is by assembly of synthetic oligonucleotides produced in a conventional, automated, oligonucleotide synthesizer.

A polynucleotide probe or primer may be labeled so that it is detectable in a variety of detection systems, including, but not limited, to enzyme (e.g., ELISA, as well as enzyme-based histochemical assays), fluorescent, radioactive, chemical, and luminescent systems. A polynucleotide probe or primer may further include a quencher moiety that, when placed in proximity to a label (e.g., a fluorescent label), causes there to be little or no signal from the label. Detection of the label may be performed by direct or indirect means (e.g., via a biotin/avidin or a biotin/stretpavidin linkage). It is not intended that the present invention be limited to any particular detection system or label.

The isolated polynucleotide may be primer that hybridizes, under stringent conditions, adjacent, upstream, or downstream to a variation in the non-coding region of the HTRA1 gene that is correlated with the occurrence of AMD in humans. The isolated polynucleotide may hybridize, under stringent conditions, to a nucleic acid molecule comprising all or a portion of a variant HTRA1 gene that is correlated with the occurrence of AMD in humans. Alternatively, the isolated polynucleotide primer may hybridize, under stringent conditions, to a nucleic acid molecule comprising at least 50 contiguous nucleotides of a variant HTRA1 gene that is correlated with the occurrence of AMD in humans. For example, a polynucleotide primer can hybridize adjacent, upstream, or downstream to the region of the human HTRA1 gene that encodes a change at position -512 from the putative transcription start site of the promoter of the human HTRA1 gene, which is identified as rs 11200638.

As used herein, the term "primer" refers to a polynucleotide that is capable of acting as a point of initiation of nucleic acid synthesis when placed under conditions in which synthesis of a primer extension product that is complementary to a nucleic acid strand occurs (for example, in the presence of nucleotides, an inducing agent such as DNA polymerase, and suitable temperature, pH, and electrolyte concentration). Alternatively, the primer may be capable of ligating to a proximal nucleic acid when placed under conditions in which ligation of two unlinked nucleic acids occurs (for example, in the presence of a proximal nucleic acid, an inducing agent such as DNA ligase, and suitable temperature, pH, and electrolyte concentration). A polynucleotide primer may be naturally occurring, as in a purified restriction digest, or may be produced synthetically. The primer is preferably single stranded for maximum efficiency in amplification, but may alternatively be double stranded. If double stranded, the primer is first treated to separate its strands before being used. Preferably, the primer is an oligodeoxyribonucleotide. The exact lengths of the primers will depend on many factors, including temperature, source of primer and the use of the method. In certain embodiments, the polynucleotide primer of the invention is at least 10 nucleotides long and hybridizes to one side or another of a variation in the non-coding region of the HTRA1 gene that is correlated with the occurrence of AMD in humans. The subject polynucleotides may contain alterations, such as one or more nucleotide substitutions, additions or deletions, provided they hybridize to their target variant HTRA1 gene with the same degree of specificity.

Described herein is a pair of primers that specifically detect a variation in the non-coding region of the HTRA1 gene that is correlated with the occurrence of AMD in humans, such as the variation identified as SNP rs 11200638. In such a case, the first primer hybridizes upstream from the variation and a second primer hybridizes downstream from the variation. It is understood that one of the primers hybridizes to one strand of a region of DNA that comprises a variation in the non-coding region of the HTRA1 gene that is correlated with the occurrence of AMD, and the second primer hybridizes to the complementary strand of a region of DNA that comprises a variation in the non-coding region of the HTRA1 gene that is correlated with the occurrence of AMD in humans. As used herein, the term "region of DNA" refers to a sub-chromosomal length of DNA.

Also described herein is an allele-specific primer that hybridizes to a site on target DNA that overlaps a variation in the non-coding region of the HTRA1 gene that is correlated with the occurrence of AMD in humans. An allele-specific primer of the invention only primes amplification of an allelic form to which the primer exhibits perfect complementarity. This primer may be used, for example, in conjunction with a second primer which hybridizes at a distal site. Amplification can thus proceed from the two primers, resulting in a detectable .product that indicates the presence of a variant HTRA1 gene that is correlated with the occurrence of AMD in humans.

### 3. Detection assays

Described herein are polynucleotides useful for detecting a variation in the non-coding region of the HTRA1 gene that is correlated with the occurrence of age related macular degeneration, such as the variation identified as SNP rs 11200638. Preferably, these polynucleotides are capable of hybridizing under stringent hybridization conditions to a region of DNA that comprises a variation in the non-coding region, for example the promoter region of the HTRA1 gene that is correlated with the occurrence of age related macular degeneration.

The polynucleotides of the invention may be used in any assay that permits detection of a variation in the non-coding region of the human HTRA1 gene that is correlated with the occurrence of AMD. Such methods may encompass, for example, DNA sequencing, hybridization, ligation, or primer extension methods. Furthermore, any combination of these methods may be utilized in the invention. In one embodiment, the presence of a variation in the non-coding region of the human HTRA1 gene that is correlated with the occurrence of AMD is detected and/or determined by DNA sequencing. DNA sequence determination may be performed by standard methods such as dideoxy chain termination technology and gel-electrophoresis, or by other methods such as by pyrosequencing (Biotage AB, Uppsala, Sweden). For example, DNA sequencing by dideoxy chain termination may be performed using unlabeled primers and labeled (e.g., fluorescent or radioactive) terminators. Alternatively, sequencing may be performed using labeled primers and unlabeled terminators. The nucleic acid sequence of the DNA in the sample can be compared to the nucleic acid sequence of wildtype DNA to identify whether a variation in the non-coding region of the HTRA1 gene that is correlated with the occurrence of AMD is present.

The presence of a variation in the non-coding region of the HTRA1 gene that is correlated with the occurrence of AMD may be detected and/or determined by hybridization. A polynucleotide probe may hybridize to a variation in the non-coding region of the HTRA1 gene, and flanking nucleotides, that is correlated with AMD, but not to a wildtype HTRA1 gene. The polynucleotide probe may comprise nucleotides that are fluorescently, radioactively, or chemically labeled to facilitate detection of hybridization. Hybridization may be performed and detected by standard methods known in the art, such as by Northern blotting, Southern blotting, fluorescent in situ hybridization (FISH), or by hybridization to polynucleotides immobilized on a solid support, such as a DNA array or microarray. As used herein, the term "DNA array," and "microarray" refers to an ordered arrangement ofhybridizable array elements. The array elements are arranged so that there are preferably at least one or more different array elements immobilized on a substrate surface. The hybridization signal from each of the array elements is individually distinguishable. In a preferred embodiment, the array elements comprise polynucleotides, although the present invention could also be used with cDNA or other types of nucleic acid array elements.

The polynucleotide probe may be used to hybridize genomic DNA by FISH. FISH can be used, for example, in metaphase cells, to detect a deletion in genomic DNA. Genomic DNA is denatured to separate the complimentary strands within the DNA double helix structure. The polynucleotide probe is then added to the denatured genomic DNA. If a variation in the non-coding region of the HTRA1 gene that is correlated with the occurrence of AMD is present, the probe will hybridize to the genomic DNA. The probe signal (e.g., fluorescence) can then be detected through a fluorescent microscope for the presence of absence of signal. The absence of signal, therefore, indicates the absence of a variation in the non-coding region of the HTRA1 gene that is correlated with the occurrence of AMD. An example of such a variation is a nucleotide base other than AG at position -512 relative to the putative transcription start site of the human HTRA1 gene. A labeled polynucleotide probe may also be applied to immobilized polynucleotides on a DNA array. Hybridization may be detected, for example, by measuring the intensity of the labeled probe remaining on the DNA array after washing. The polynucleotides described herein may also be used in commercial assays, such as the Taqman assay (Applied Biosystems, Foster City, CA).

The presence of a variation in the non-coding region of the human HTRA1 gene that is correlated with the occurrence of AMD may be detected and/or determined by primer extension with DNA polymerase. A polynucleotide primer of the invention may hybridize immediately adjacent to the variation. A single base sequencing reaction using labeled dideoxynucleotide terminators may be used to detect the variation. The presence of a variation will result in the incorporation of the labeled terminator, whereas the absence of a variation will not result in the incorporation of the terminator. A polynucleotide primer of the invention may hybridize to a variation in the non-coding region of the HTRA1 gene that is correlated with the occurrence of AMD. The primer, or a portion thereof, will not hybridize to a wildtype HTRA1 gene. The presence of a variation will result in primer extension, whereas the absence of a variation will not result in primer extension. The primers and/or nucleotides may further include fluorescent, radioactive, or chemical probes. A primer labeled by primer extension may be detected by measuring the intensity of the extension product, such as by gel electrophoresis, mass spectrometry, or any other method for detecting fluorescent, radioactive, or chemical labels.

The presence of a variation in the non-coding region of the HTRA1 gene that is correlated with the occurrence of AMD may be detected and/or determined by ligation. A polynucleotide primer of the invention may hybridize to a variation in the non-coding region of the human HTRA1 gene that is correlated with the occurrence of AMD, such as the variation that is identified as SNP rs 11200638. The primer, or a portion thereof will not hybridize to a wildtype HTRA1 gene. A second polynucleotide that hybridizes to a region of the HTRA1 gene immediately adjacent to the first primer is also provided. One, or both, of the polynucleotide primers may be fluorescently, radioactively, or chemically labeled. Ligation of the two polynucleotide primers will occur in the presence of DNA ligase if a variation in the non-coding region of the HTRA1 gene that is correlated with the occurrence of AMD is present. Ligation may be detected by gel electrophoresis, mass spectrometry, or by measuring the intensity of fluorescent, radioactive, or chemical labels.

The presence of a variation in the non-coding region of the human HTRA1 gene that is correlated with the occurrence of AMD may be detected and/or determined by single-base extension (SBE). For example, a fluorescently-labeled primer that is coupled with fluorescence resonance energy transfer (FRET) between the label of the added base and the label of the primer may be used. Typically, the method, such as that described by Chen et al., (PNAS 94:10756-61 (1997), incorporated herein by reference) uses a locus-specific polynucleotide primer labeled on the 5' terminus with 5-carboxyfluorescein (FAM). This labeled primer is designed so that the 3' end is immediately adjacent to the polymorphic site of interest. The labeled primer is hybridized to the locus, and single base extension of the labeled primer is performed with fluorescently labeled dideoxyribonucleotides (ddNTPs) in dye-terminator sequencing fashion, except that no deoxyribonucleotides are present. An increase in fluorescence of the added ddNTP in response to excitation at the wavelength of the labeled primer is used to infer the identity of the added nucleotide.

Methods of detecting a variation in the non-coding region of the HTRA1 gene that is correlated with the occurrence of AMD may include amplification of a region of DNA that comprises the variation. Any method of amplification may be used. For example, a region of DNA comprising the variation is amplified by using polymerase chain reaction (PCR). PCR was initially described by Mullis (See e.g., U.S. Pat. Nos. 4,683,195 4,683,202, and 4,965,188, herein incorporated by reference), which describes a method for increasing the concentration of a region of DNA, in a mixture of genomic DNA, without cloning or purification. Other PCR methods may also be used for nucleic acid amplification, including but not limited to RT-PCR, quantitative PCR, real time PCR, Rapid Amplified Polymorphic DNA Analysis, Rapid Amplification of cDNA Ends (RACE), or rolling circle amplification. For example, the polynucleotide primers of the invention are combined with a DNA mixture (or any polynucleotide sequence that can be amplified with the polynucleotide primers of the invention), wherein the DNA comprises the HTRA1 gene. The mixture also includes the necessary amplification reagents (e.g., deoxyribonucleotide triphosphates, buffer, etc.) necessary for the thermal cycling reaction. According to standard PCR methods, the mixture undergoes a series of denaturation, primer annealing, and polymerase extension steps to amplify the region of DNA that comprises the variation in the non-coding region of the HTRA1 gene. An example for such a variation is the presence of a nucleotide base other than G at position -512 relative to the putative transcription start site of the human HTRA1 gene. The length of the amplified region of DNA is determined by the relative positions of the primers with respect to each other, and therefore, this length is a controllable parameter. For example, hybridization of the primers may occur such that the ends of the primers proximal to the variation are separated by 1 to 10,000 base pairs (e.g., 10 base pairs (bp) 50 bp, 200 bp, 500 bp, 1,000 bp, 2,500 bp, 5,000 bp, or 10,000 bp).

Standard instrumentation known to those skilled in the art are used for the amplification and detection of amplified DNA. For example, a wide variety of instrumentation has been developed for carrying out nucleic acid amplifications, particularly PCR, e.g. Johnson et al, U.S. Pat. No. 5,038,852 (computer-controlled thermal cycler); Wittwer et al, Nucleic Acids Research, 17: 4353-4357 (1989)(capillary tube PCR); Hallsby, U.S. Pat. No. 5,187,084 (air-based temperature control); Garner et al, Biotechniques, 14: 112-115 (1993)(high-throughput PCR in 864-well plates); Wilding et al, International application No. PCT/US93/04039 (PCR in micro-machined structures); Schnipelsky et al, European patent application No. 90301061.9 (publ. No. 0381501 A2)(disposable, single use PCR device), and the like. Real-time PCR or other methods known in the art such as the Taqman assay may be utilized.

A variant HTRA1 gene that is correlated with the occurrence of AMD in humans may be detected using single-strand conformation polymorphism analysis, which identifies base differences by alteration in electrophoretic migration of single stranded PCR products, as described in Orita et al., Proc. Nat. Acad. Sci. 86, 2766-2770 (1989). Amplified PCR products can be generated as described above, and heated or otherwise denatured, to form single stranded amplification products. Single-stranded nucleic acids may refold or form secondary structures which are partially dependent on the base sequence. The different electrophoretic mobilities of single-stranded amplification products can be related to base-sequence differences between alleles of target sequences.

The amplified DNA may be analyzed in conjunction with one of the detection methods described herein, such as by DNA sequencing. The amplified DNA may alternatively be analyzed by hybridization with a labeled probe, hybridization to a DNA array or microarray, by incorporation of biotinylated primers followed by avidin-enzyme conjugate detection, or by incorporation of ³²P-labeled deoxynucleotide triphosphates, such as dCTP or dATP, into the amplified segment. In a specific embodiment, the amplified DNA is analyzed by determining the length of the amplified DNA by electrophoresis or chromatography. For example, the Amplified DNA is analyzed by gel electrophoresis. Methods of gel electrophoresis are well known in the art. See for example, Current Protocols in Molecular Biology, eds. Ausubel et al., John Wiley & Sons: 1992. The amplified DNA can be visualized, for example, by fluorescent or radioactive means, or with other dyes or markers that intercalate DNA. The DNA may also be transferred to a solid support such as a nitrocellulose membrane and subjected to Southern Blotting following gel electrophoresis. The DNA may be exposed to ethidium bromide and visualized under ultra-violet light.

### Antibodies

By using immunogens derived from the HTRA1 peptide, anti-protein/anti-peptide antisera or monoclonal antibodies can be made by standard protocols (see, for example, Antibodies: A Laboratory Manual ed. by Harlow and Lane (Cold Spring Harbor Press: 1988)). A mammal, such as a mouse, a hamster or rabbit can be immunized with an immunogenic form of an HTRA1 peptide, an antigenic fragment which is capable of eliciting an antibody response, or a fusion protein. The inoculated mouse may not express endogenous HTRA1, thus facilitating the isolation of antibodies that would otherwise be eliminated as anti-self antibodies. Techniques for conferring immunogenicity on a protein or peptide include conjugation to carriers or other techniques well known in the art. An immunogenic portion of a HTRA1 peptide can be administered in the presence of adjuvant. The progress of immunization can be monitored by detection of antibody titers in plasma or serum. Standard ELISA or other immunoassays can be used with the immunogen as antigen to assess the levels of antibodies.

Following immunization of an animal with an antigenic preparation of a HTRA1 polypeptide, antisera can be obtained and, if desired, polyclonal antibodies can be isolated from the serum. To produce monoclonal antibodies, antibody-producing cells (lymphocytes) can be harvested from an immunized animal and fused by standard somatic cell fusion procedures with immortalizing cells such as myeloma cells to yield hybridoma cells. Such techniques are well known in the art, and include, for example, the hybridoma technique (originally developed by Kohler and Milstein, (1975) Nature, 256: 495-497), the human B cell hybridoma technique (Kozbar et al., (1983) Immunology Today, 4: 72), and the EBV-hybridoma technique to produce human monoclonal antibodies (Cole et al., (1985) Monoclonal Antibodies and Cancer Therapy, Alan R. Liss, Inc. pp. 77-96). Hybridoma cells can be screened immunochemically for production of antibodies specifically reactive with HTRA1 polypeptide and monoclonal antibodies isolated from a culture comprising such hybridoma cells.

The term "antibody" as used herein is intended to include fragments thereof which are also specifically reactive with HTRA1 polypeptide. Antibodies can be fragmented using conventional techniques and the fragments screened for utility in the same manner as described above for whole antibodies. For example, F(ab)₂ fragments can be generated by treating antibody with pepsin. The resulting F(ab)₂ fragment can be treated to reduce disulfide bridges to produce Fab fragments. The antibodies described herein include bispecific, single-chain, and chimeric and humanized molecules having affinity for HTRA1 polypeptide conferred by at least one CDR region of the antibody. The antibody may further comprise a label attached thereto and able to be detected (e.g., the label can be a radioisotope, fluorescent compound, enzyme or enzyme co-factor).

An antibody may be a monoclonal antibody, and described herein are methods for generating novel antibodies that bind specifically to HTRA1 polypeptides. For example, a method for generating a monoclonal antibody that binds specifically to HTRA1 polypeptide may comprise administering to a mouse an amount of an immunogenic composition comprising the HTRA1 polypeptide effective to stimulate a detectable immune response, obtaining antibody-producing cells (e.g., cells from the spleen) from the mouse and fusing the antibody-producing cells with myeloma cells to obtain antibody-producing hybridomas, and testing the antibody-producing hybridomas to identify a hybridoma that produces a monocolonal antibody that binds specifically to HTRA1 polypeptide. Once obtained, a hybridoma can be propagated in a cell culture, optionally in culture conditions where the hybridoma-derived cells produce the monoclonal antibody that binds specifically to the HTRA1 polypeptide. The monoclonal antibody may be purified from the cell culture.

Antibodies reactive to HTRA1 are commercially available (for example from Imgenex) and are also described in, for example, PCT application No. WO 00/08134.

The term "specifically reactive with" as used in reference to an antibody is intended to mean, as is generally understood in the art, that the antibody is sufficiently selective between the antigen of interest (e.g., a HTRA1 polypeptide) and other antigens that are not of interest that the antibody is useful for, at minimum, detecting the presence of the antigen of interest in a particular type of biological sample. In certain methods employing the antibody, such as therapeutic applications, a higher degree of specificity in binding may be desirable. Monoclonal antibodies generally have a greater tendency (as compared to polyclonal antibodies) to discriminate effectively between the desired antigens and cross-reacting polypeptides. One characteristic that influences the specificity of an antibody-antigen interaction is the affinity of the antibody for the antigen. Although the desired specificity may be reached with a range of different affnities, generally preferred antibodies will have an affinity (a dissociation constant) of about 10⁻⁶, 10⁻⁷ 10⁻⁸, 10⁻⁹ or less.

In addition, the techniques used to screen antibodies in order to identify a desirable antibody may influence the properties of the antibody obtained. For example, if an antibody is to be used for binding an antigen in solution, it may be desirable to test solution binding. A variety of different techniques are available for testing interaction between antibodies and antigens to identify particularly desirable antibodies. Such techniques include ELISAs, surface plasmon resonance binding assays (e.g., the BIAcore binding assay, BIAcore AB, Uppsala, Sweden), sandwich assays (e.g., the paramagnetic bead system of IGEN International, Inc., Gaithersburg, Maryland), western blots, immunoprecipitation assays, and immunohistochemistry.

### 6. Pharmaceutical Compositions

The methods and compositions described herein for treating a subject suffering from AMD may be used for the prophylactic treatment of individuals who have been diagnosed or predicted to be at risk for developing AMD. In this case, the composition is administered in an amount and dose that is sufficient to delay, slow, or prevent the onset of AMD or related symptoms. Alternatively, the methods and compositions described herein may be used for the therapeutic treatment of individuals who suffer from AMD. In this case, the composition is administered in an amount and dose that is sufficient to delay or slow the progression of the condition, totally or partially, or in an amount and dose that is sufficient to reverse the condition to the point of eliminating the disorder. It is understood that an effective amount of a composition for treating a subject who has been diagnosed or predicted to be at risk for developing AMD is a dose or amount that is in sufficient quantities to treat a subject or to treat the disorder itself.

The compositions may be formulated with a pharmaceutically acceptable carrier, or for administration in any convenient way for use in human medicine.

Therapeutic methods may include administering the composition topically, systemically, or locally. The composition may be administered locally in the eye that is affected or in risk of being affected by AMD. For example, therapeutic compositions of the invention may be formulated for administration by, for example, injection (e.g., intravenously, subcutaneously, or intramuscularly), inhalation or insufflation (either through the mouth or the nose) or oral, buccal, sublingual, transdermal, nasal, or parenteral administration. In another specific embodiment local administration can be further restricted to the area in the eye that is affected by AMD such as the area between the retinal pigment epithelium (RPE) and Bruch's membrane, for example by targeted injection of the therapeutic composition. The compositions described herein may be formulated as part of an implant or device. When administered, the therapeutic composition for use in this invention is in a pyrogen-free, physiologically acceptable form. Further, the composition may be encapsulated or injected in a viscous form for delivery to the site where the target cells are present, such as to the cells of the eye. Techniques and formulations generally may be found in Remington's Pharmaceutical Sciences, Meade Publishing Co., Easton, PA. In addition to SRF, AP2 alpha, or HTRA1 polypeptide or a nucleic acid molecule coding for a SRF, AP2 alpha, or HTRA1 polypeptide, or variant thereof, therapeutically useful agents may optionally be included in any of the compositions as described above. Furthermore, therapeutically useful agents may, alternatively or additionally, be administered simultaneously or sequentially with SRF, AP2 alpha, or HTRA1 polypeptide or a nucleic acid molecule coding for a SRF, AP2 alpha, or HTRA1 polypeptide, or variant thereof according to the methods of the invention. In addition combinations including a CFH polypeptide or a nucleic acid molecule coding for a CFH polypeptide, or variant thereof, are contemplated.

Compositions of the invention can be administered orally, e.g., in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of an agent as an active ingredient. An agent may also be administered as a bolus, electuary or paste.

In solid dosage forms for oral administration (capsules, tablets, pills, dragees, powders, granules, and the like), one or more therapeutic compounds may be mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose, and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

Liquid dosage forms for oral administration include pharmaceutically acceptable emulsions, microemulsions, solutions, suspensions, syrups, and elixirs. In addition to the active ingredient, the liquid dosage forms may contain inert diluents commonly used in the art, such as water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor, and sesame oils), glycerol, tetrahydrofuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, coloring, perfuming, and preservative agents.

Suspensions, in addition to the active compounds, may contain suspending agents such as ethoxylated isostearyl alcohols, polyoxyethylene sorbitol, and sorbitan esters, microcrystalline cellulose, aluminum metahydroxide, bentonite, agar-agar and tragacanth, and mixtures thereof.

Certain compositions disclosed herein may be administered topically, either to skin or to mucosal membranes. The topical formulations may further include one or more of the wide variety of agents known to be effective as skin or stratum corneum penetration enhancers. Examples of these are 2-pyrrolidone, N-methyl-2-pyrrolidone, dimethylacetamide, dimethylformamide, propylene glycol, methyl or isopropyl alcohol, dimethyl sulfoxide, and azone. Additional agents may further be included to make the formulation cosmetically acceptable. Examples of these are fats, waxes, oils, dyes, fragrances, preservatives, stabilizers, and surface active agents. Keratolytic agents such as those known in the art may also be included. Examples are salicylic acid and sulfur.

Dosage forms for the topical or transdermal administration include powders, sprays, ointments, pastes, creams, lotions, gels, solutions, patches, and inhalants. The active compound may be mixed under sterile conditions with a pharmaceutically acceptable carrier, and with any preservatives, buffers, or propellants which may be required. The ointments, pastes, creams and gels may contain, in addition to a subject compound of the invention (e.g., an isolated or recombinantly produced nucleic acid molecule coding for SRF, AP2 alpha or HTRA1 polypeptide or an isolated or recombinantly produced SRF, AP2, HTRA1 polypeptide, or variant thereof, such as a dominant negative variant), excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a subject compound, excipients such as lactose, talc, silicic acid, aluminum hydroxide, calcium silicates, and polyamide powder, or mixtures of these substances. Sprays can additionally contain customary propellants, such as chlorofluorohydrocarbons and volatile unsubstituted hydrocarbons, such as butane and propane.

It is understood that the dosage regimen will be determined for an individual, taking into consideration, for example, various factors which modify the action of the subject compounds of the invention (e.g., an isolated or recombinantly produced nucleic acid molecule coding for SRF, AP2 alpha or HTRA1 polypeptide or an isolated or recombinantly produced SRF, AP2, HTRA1 polypeptide, or variant thereof, such as a dominant negative variant), the severity or stage of AMD, route of administration, and characteristics unique to the individual, such as age, weight, and size. A person of ordinary skill in the art is able to determine the required dosage to treat the subject. In one embodiment, the dosage can range from about 1.0 ng/kg to about 100 mg/kg body weight of the subject. Based upon the composition, the dose can be delivered continuously, or at periodic intervals. For example, on one or more separate occasions. Desired time intervals of multiple doses of a particular composition can be determined without undue experimentation by one skilled in the art. For example, the compound may be delivered hourly, daily, weekly, monthly, yearly (e.g. in a time release form) or as a one time delivery.

In certain embodiments, pharmaceutical compositions suitable for parenteral administration may comprise SRF, AP2 alpha or HTRA1 polypeptide or a nucleic acid molecule coding for SRF, AP2 alpha or HTRA1 polypeptide, or variant thereof, such as a dominant negative variant, in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents. Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

The compositions of the invention may also contain adjuvants, such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption, such as aluminum monostearate and gelatin.

A person of ordinary skill in the art is able to determine the required amount to treat the subject. It is understood that the dosage regimen will be determined for an individual, taking into consideration, for example, various factors which modify the action of the subject compounds of the invention, the severity or stage of AMD, route of administration, and characteristics unique to the individual, such as age, weight, and size. A person of ordinary skill in the art is able to determine the required dosage to treat the subject. In one embodiment, the dosage can range from about 1.0 ng/kg to about 100 mg/kg body weight of the subject. The dose can be delivered continuously, or at periodic intervals. For example, on one or more separate occasions. Desired time intervals of multiple doses of a particular composition can be determined without undue experimentation by one skilled in the art. For example, the compound may be delivered hourly, daily, weekly, monthly, yearly (e.g. in a time release form) or as a one time delivery. As used herein, the term "subject" means any individual animal capable of becoming afflicted with AMD. The subjects include, but are not limited to, human beings, primates, horses, birds, cows, pigs, dogs, cats, mice, rats, guinea pigs, ferrets, and rabbits. In the preferred embodiment, the subject is a human being.

Samples used in the methods described herein may comprise cells from the eye, ear, nose, teeth, tongue, epidermis, epithelium, blood, tears, saliva, mucus, urinary tract, urine, muscle, cartilage, skin, or any other tissue or bodily fluid from which sufficient DNA or RNA can be obtained.

The sample should be sufficiently processed to render the DNA or RNA that is present available for assaying in the methods described herein. For example, samples may be processed such that DNA from the sample is available for amplification or for hybridization to another polynucleotide. The processed samples may be crude lysates where available DNA or RNA is not purified from other cellular material. Alternatively, samples may be processed to isolate the available DNA or RNA from one or more contaminants that are present in its natural source. Samples may be processed by any means known in the art that renders DNA or RNA available for assaying in the methods described herein. Methods for processing samples may include, without limitation, mechanical, chemical, or molecular means of lysing and/or purifying cells and cell lysates. Processing methods may include, for example, ion-exchange chromatography, size exclusion chromatography, affinity chromatography, hydrophobic interaction chromatography, gel filtration chromatography, ultrafiltration, electrophoresis, and immunoaffinity purification with antibodies specific for particular epitopes of the polypeptide.

### 8. Kits

Also provided herein are kits, e.g., kits for therapeutic purposes or kits for detecting a variant HTRA1 gene in a sample from an individual. In one embodiment, a kit comprises at least one container means having disposed therein a premeasured dose of a polynucleotide probe that hybridizes, under stringent conditions, to a variation in the non-coding region of the HTRA1 gene that is correlated with the occurrence of AMD in humans. In another embodiment, a kit comprises at least one container means having disposed therein a premeasured dose of a polynucleotide primer that hybridizes, under stringent conditions, adjacent to one side of a variation in the non-coding region of the HTRA1 gene that is correlated with the occurrence of AMD in humans. In a further embodiment, a second polynucleotide primer that hybridizes, under stringent conditions, to the other side of a variation in the non-coding region of the HTRA1 gene that is correlated with the occurrence of AMD in humans is provided in a premeasured dose. Kits further comprise a label and/or instructions for the use of the therapeutic or diagnostic kit in the detection of HTRA1 in a sample. Kits may also include packaging material such as, but not limited to, ice, dry ice, styrofoam, foam, plastic, cellophane, shrink wrap, bubble wrap, paper, cardboard, starch peanuts, twist ties, metal clips, metal cans, drierite, glass, and rubber (see products available from www.papermart.com. for examples of packaging material). In yet another embodiment the polynucleotide probe that hybridizes, under stringent conditions, to a variation in the non-coding region of the HTRA1 gene that is correlated with the occurrence of AMD in humans is combined with a second polynucleotide probe that hybridizes, under stringent conditions, to a variation in the CFH gene that is correlated with the occurrence of AMD in humans.

The practice of the present methods will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning: A Laboratory Manual, Cold Spring Harbor Laboratory (2001); DNA Cloning, Volumes I and II (D. N. Glover ed., 1985); Oligonucleotide Synthesis (M. J. Gait ed., 1984); Mullis et al. U.S. Patent No: 4,683,195; Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Manipulating the Mouse Embryo, (Cold Spring Harbor Laboratory Press, Cold Spring Harbor, N.Y., 1986).

### EXAMPLES

The following examples are for illustrative purposed and not intended to be limiting in any way.

The following Methods and Materials were used in the work described herein, particularly in Examples 1 and 2.

### Study participants

Both previously (L. Baum et al., Ophthalmologica 217, 111 (2003), C. P. Pang et al., Ophthalmologica 214, 289 (2000)) and newly recruited participants were used in this study. All recruitment was carried out according to the criteria described in (C. P. Pang et al., Ophthalmologica 214, 289 (2000)). Briefly, all participants received a standard examination protocol and visual-acuity measurement. Slitlamp biomicroscopy of the fundi was performed by an experienced ophthalmologist, and stereoscopic color fundus photographs were taken by a trained ophthalmic photographer. Grading was carried out using the standard classification suggested by the International Age related Maculopathy Epidemiological Study Group. Controls showed no sign of AMD or any other major eye diseases except senile cataracts. During history taking, participants were asked about their smoking habits and that information was recorded. A smoker was defined as a person who smoked at least 5 cigarettes daily for more than one year. Smokers were subdivided into three groups: those who had never smoked, those who were ex-smokers, and those who were current smokers.

Of the 117 cases available to Applicants, Applicants excluded any classified as being at AMD stage 3 or 4 (n=18) to select only the "wet" cases of AMD. To more closely match the age distribution between cases and controls, Applicants excluded cases > 90 years of age (n=2). Because the original control population (n=153) was significantly younger than the cases, Applicant excluded cases < 65 years of age (n=22). The characteristics of the final group of 96 cases and 130 controls are given in Table 1.

**Table 1. Characteristics of cases and controls in the Hong Kong cohort.**

| | Cases (AMD Grade 5) | Controls |
|---|---|---|
| Total | 96 | 130 |
| Mates (%) | 68 | 33 |
| | | |
| Mean age (± s.d.) (years) | 74.9±6.8 | 74.2±5.7 |
| Age range (years) | 60-89 | 65-99 |
| | | |
| Smokers (%) | 63 | 26 |

### Genotyping

Applicant genotyped each individual using the Affymetrix GeneChip Mapping 100K Set of microarrays. The SNP genotyping assay consisted of two chips (XbaI and HindIII) with 58,960 and 57,244 SNPs, respectively. Approximately 250ng of genomic DNA was processed for each chip according to the Affymetrix protocol (H. Matsuzaki et al., Nat Methods 1, 109 (2004)).

Applicant deemed only those individual chips achieving a call rate of >90% to be usable for analysis. 268 individuals were genotyped for HindIII and 266 for HindIII and XbaI.

Individual autosomal SNP data quality was assessed by examining the call rates. SNPs with call rates < 85% were eliminated from the analysis. To further eliminate SNPs with possible genotyping errors, Applicant excluded heterozygous SNPs without any observed heterozygotes and SNPs with only heterozygotes. To eliminate uninformative SNPs, applicant excluded non-heterozygous SNPs. Finally, deviations from Hardy Weinberg Equilibrium (HWE) were assessed, and Applicant excluded SNPs with a HWE χ² > 50. Through these exclusions, largely due to low call rates of <85%, 97,824 autosomal SNPs remained for analysis. These data are summarized in Table 2.

**Table 2. Genotyping data quality.**

| *Number of Individuals* | |
|---|---|
| Hind | 268 |
| Xba | 267* |
| Per-chip data quality | |
| Median call rate per chip (Hind) | 99.41 % |
| Median call rate per chip (Xba) | 99.33% |
| Minimum call rate per chip (Hind) | 94.33% |
| Minimum call rate per chip (Xba) | 76.72%* |
| Per-individual data quality | |
| Average number of matches for common SNPs between two chips^{#} | 30.7 |
| Minimum number of matches for common SNPs between two chips^{#} | 26 |
| Total Number of SNPs | 116204 |
| Number of Autosomal SNPs | 113841 |
| Call rate (per-SNP) | |
| SNPs with 100% call rate | 71156 |
| SNPs with call rate between 85% and 100% | 41934 |
| SNPs with call rate less than 85% | 751 |
| SNPs with call rate above 85% (Hind; 40 or less NoCalls) | 113090 |
| Locus Polymorphism (for autosomal SNPs with call rates > 85%) | |
| Number of SNPs with no polymorphism observed | 14867 |
| Number of SNPs with only heterozygotes observed | 17 |
| Number of polymorphic SNPs with no heterozygotes observed | 36 |
| Number of SNPs with minor allele frequency < 0.01 | 6008 |
| Hardy-Weinberg Equilibrium (for polymorphic SNPs regardless of MAF) | |
| Number of SNPs with HWE X² > 50 | 346 |
| Final number of SNPs | 97824 |

| | |
|---|---|
| *After the one Xbal chip with the low call rate was removed there were 266 samples genotyped on the Xbal chip and the minimum call rate was 95.85%. ^{#}Out of 31 SNPs that are in common between the two chips. | |

### Statistical Analysis

The initial analysis was carried out by constructing 2 x 2 tables of the allele counts and 2 x 3 tables of the genotype counts for each SNP in all cases and controls. Subsequently, Pearson's χ² statistics were calculated and P-values computed by comparing the χ² statistic to a χ² distribution with 1 or 2 df for the allelic and genotypic tests, respectively. SNPs yielding a P-value smaller than 5.1 x 10⁻⁷ (Bonferroni adjusted significance of 0.05 [0.05/97,824]) were selected for further analysis.

Two differerent methods were used, Genomic Control (GC) and Genomic Control, F-test (GCF) H. Okamoto et al., Mol Vis 12, 156 (2006) to test for the presence of admixture in the sample. The first method, GC, uses the median of the χ² values for a number of unassociated SNPs (null SNPs) in the study. For the pupose of the genomic control tests, all non-significant SNPs were considered to be null SNPs. Then, the χ² value was divided by the median and compared to a χ² distribution to test for significance. The second method, GCF, uses the mean of the null χ² values instead of the median. The individual χ² values are again divided by the mean and the resulting statistic is compared to an F distribution with 1, L degrees of freedom, where L is the number of null SNPs used to compute the mean.

Odds ratios, population attributable risks, and their respective confidence intervals were calculated using standard formulae P. Armitage, G. Berry, Statistical Methods in Medical Research (Balckwell Scientific Publications, 1971). Because of the relatively high frequency of the risk allele at rs 10490924 in the case/control sample (55%), the corresponding attributable risk will be overestimated, and so does not provide a good estimate of the risk in the population.

To identify the region of interest around rs10490924, Applicant examined the region bounded by pairwise SNPs in which all four gametes were observed R. R. Hudson, N. L. Kaplan, Genetics 111, 147 (1985), subsequently referred to as the "4-gamete region." Applicant examined the pattern of linkage disequilibrium (LD) by constructing haplotypes for the seven internal SNPs in the 4-gamete region using the SNPHAP D. Clayton. http://www-gene.cimr.cam.ac.uk/clayton/software and the PHASE M. Stephens, N. J. Smith, P. Donnelly, Am JHum Genet 68, 978 (2001) algorithms. Both algorithms yielded the same haplotypes in similar frequencies. Once haplotypes were reconstructed, D', a standard measure of LD, was calculated using the Haploxt program G. R. Abecasis, W. O. Cookson, Bioinformatics 16, 182 (2000). LD patterns for the combined case/control sample were then visualized using GOLD (R. Klein et al, Ophrhalmology 113, 373 (2006)). Estimated haplotype frequencies for the case and control groups combined and for each separate group are given in Table 3.

**Table 3. Haplotype analysis of seven SNPs: rs2421019, rs2292623, rs2292625, rs10510110, rs2280141, rs2736911, rs10490924 in the 4 gamete region. Haplotype frequency estimates as determined by PHASE for the entire population "all" and for the case and control populations separately.**

| | TIC | A/G | A/G | T/C | T/G | T/C | T/G | All | Case | Control |
|---|---|---|---|---|---|---|---|---|---|---|
| N1 | 2 | 1 | 2 | 2 | 2 | 2 | 1 | 0.45743 | 0.61100 | 0.34024 |
| N2 | 2 | 1 | 2 | 2 | 2 | 2 | 2 | 0.17255 | 0.09849 | 0.22908 |
| N3 | 2 | 2 | 2 | 1 | 1 | 1 | 2 | 0.14944 | 0.12047 | 0.17154 |
| N4 | 1 | 1 | 2 | 1 | 1 | 2 | 2 | 0.07105 | 0.03783 | 0.09640 |
| N5 | 1 | 1 | 2 | 1 | 1 | 2 | 1 | 0.05739 | 0.05845 | 0.05659 |
| N6 | 1 | 1 | 1 | 1 | 1 | 2 | 2 | 0.03777 | 0.03009 | 0.04363 |
| N7 | 2 | 2 | 2 | 1 | 1 | 2 | 1 | 0.02841 | 0.02597 | 0.03026 |
| N8 | 2 | 2 | 2 | 1 | 1 | 2 | 2 | 0.01989 | 0.01302 | 0.02514 |
| N9 | 2 | 1 | 2 | 2 | 2 | 1 | 2 | 0.00364 | 0.00021 | 0.00626 |
| N10 | 2 | 2 | 2 | 2 | 2 | 2 | 1 | 0.00188 | 0.00431 | 0.00003 |
| N11 | 1 | 1 | 2 | 1 | 1 | 1 | 2 | 0.00041 | 0.00004 | 0.00068 |
| N12 | 1 | 1 | 1 | 1 | 1 | 2 | 1 | 0.00006 | 0.00006 | 0.00006 |
| N13 | 2 | 1 | 1 | 2 | 2 | 2 | 2 | 0.00002 | 0.00001 | 0.00003 |

The most probable haplotype pair for each individual was obtained from PHASE and then used to determine haplotype counts given in Table 4.

**Table 4. Haplotype counts (and frequency) for cases and controls determined by PHASE using the most probable haplotype pair as the haplotype assignment for an individual.**

| Haplotype | Case | Control |
|---|---|---|
| N1 | 132 (0.634) | 87 (0.335) |
| N2 | 17 (0.082) | 62 (0.239) |
| N3 | 24 (0.115) | 45 (0.173) |
| N4 | 9 (0.043) | 29 (0.112) |
| N6 | 6 (0.029) | 11 (0.042) |
| N5 | 13 (0.063) | 10 (0.038) |
| N7 | 5 (0.024) | 10 (0.038) |
| N8 | 1 (0.005) | 4 (0.015) |
| N11 | 0 | 1 (0.004) |
| N9 | 0 | 1 (0.004) |
| N10 | 1 (0.005) | 0 |

These haplotype counts were used to create the contingency table and to estimate the effect size of the risk haplotype given in Table 5.

**Table 5. Contingency table and effect size of the risk haplotype. For the OR and PAR, only the autosomal recessive case is considered, where cases and controls with two copies of the N1 haplotype are compared to those with zero copies.**

| | Copies of Risk Haplotype (N1) | | | | |
|---|---|---|---|---|---|
| | 2 | 1 | 0 | OR (95%Cl) | PAR (95%Cl) |
| Case | 46(0.44) | 40 (0.39) | 18 (0.17) | 10.40 (4.68-23.14) | 0.81 (0.62-0.91) |
| Control | 14(0.11) | 59 (0.45) | 57 (0.44) | | |

To investigate the pattern of LD in this region, Applicant used the publicly available HapMap database, which contains information on 45 unrelated Han Chinese individuals from Beijing (CHB). Genotypes for 183 SNPs bound by the 4-gamete region were extracted from the HapMap database. Genotypes were uploaded into Haploview (J. C. Barrett, B. Fry, J. Maller, M. J. Daly, Bioinformatics 21, 263 (2005)) to calculate LD statistics. Not all 183 of the HapMap SNPs within in this region were genotyped or passed the default quality control checks (Hardy-Weinberg *P*-value > 0.01, minimum percentage of genotyped samples > 75%, maximum of one mendelian inconsistency and a minimum allele frequency of 0.001). Haplotype blocks were identified using the parameters set forth by Gabriel et al (S. B. Gabriel et al., Science 296, 2225 (2002)), i.e., 95% confidence intervals around D' were used to determine blocks.

Among the putative recombination sites revealed by the four-gamete test to surround the marker SNP rs10490924 (R. J. Klein et al., Science 308, 385 (2005)), five major haplotypes, N1 - N5, inferred from nine SNPs (extending 63.9 kb), were identified accounting for >90% of all haplotypes in the sample. The odds ratio (OR) for two copies of the risk haplotype, N1, is 10.40, and its 95% confidence interval (CI) overlaps with that of the single SNP rs10490924, 4.68-23.14 vs. 4.83-25.69 (Table 5).LD was measured and plotted for each pair of the nine SNPs. SNP rs10490924 appears to be in LD with the upstream SNPs in *PLEKHA1,* but the next SNP genotyped is too far downstream (26.3 kb) to provide meaningful information about recombination/homoplasy breakpoints. The much denser sets of SNPs from the publicly available HapMap database for the Han Chinese in Beijing (CHB) population provided by international HapMap data (D. Altshuler et al., Nature 437, 1299 (2005)) did not resolve this matter, showing that rs10490924 was not in LD with either gene in the region in this population, and did not enable Applicants to uncover the disease-causing variant.

### Identification of a new SNP

Applicant resequenced the exons of the two genes flanking rs10490924, PLEKHA1 and HTRA1, as well as a portion of the 5' upstream sequence to capture any potential promoter variants. Applicant sequenced DNA samples from cases homozygous for the rs10490924 risk allele (TT) and controls homozygous for the non-risk allele (GG). Eighty-eight samples, 50 cases and 38 controls, were immediately available for sequencing. All sequencing steps (primer design, PCR amplification, bi-directional sequencing, and mutation analysis) were carried out by Genaissance Pharmaceuticals (New Haven, CT).

Applicant identified 43 polymorphisms in the 22 fragments that were sequenced in this region Table 6.

**Table 6. Polymorphisms identified through the resequencing of the PLEKHA1 and HTRA 1 genes. The AccPos for each polymorphism refers to the position in the GenBank accession GPI_36186.1 for PLEKHA1 and BX842242.1 for HTRA1.**

| Gene | Region | rs# | AccPos | Seq. Frag. 4850223 | Change | AA Change | Type |
|---|---|---|---|---|---|---|---|
| *PLEKHA1* | intron 2 | | 26919 | | G/A | | Noncoding |
| *PLEKHA1* | intron 2 | | 27167 | 4850223 | T/C | | Noncoding |
| *PLEKHA1* | intron 3 | rs9988734 | 29617 | 4850224 | A/G | | Noncoding |
| *PLEKHA*1 | intron 5 | | 35992 | 18578027 | -/T/() | | Noncoding |
| *PLEKHA1* | intron 6 | rs3215235 | 45061 | 4850226 | TCTAA/- | | Noncoding |
| *PLEKHA1* | intron 8 | | 53481 | 18579169 | T/G | | Noncoding |
| *PLEKHA1* | intron 9 | | 53755 | 18579169 | T/C | | Noncoding |
| *PLEKHA1* | intron 9 | rs11200624 | 53830 | 18579169 | A/G | | Noncoding |
| *PLEKHA1* | exon 10 | | 54250 | 4850228 | A/T | Tyr 268 Phe | Nonsynonomou |
| *PLEKHA1* | intron 10 | rs9783213 | 54349 | 4850228 | G/A | | Noncoding |
| *PLEKHA1* | intron 10 | rs2292625 | 56149 | 4850229 | G/A | | Noncoding |
| *PLEKHA1* | intron 11 | | 56407 | 4850229 | C/T | | Noncoding |
| *PLEKHA*1 | intron 11 | rs2292626 | 56496 | 4850229 | C/T | | Noncoding |
| *PLEKHA*1 | intron 11 | | 58895 | 4873333 | G/A | | Noncoding |
| *PLEKHA*1 | exon 12 | rs1045216 | 58979 | 4873333 | G/A | Ala 320 Thr | Nonsynonomous |
| *PLEKHA1* | exon 12 | | 59444 | 4873335 | A/G/T | | Synonomous |
| *HTRA1* | promoter | | 58157 | 710594798 | G/T | | Noncoding |
| *HTRA1* | promoter | rs11200638 | 58120 | 710594798 | A/G | | Noncoding |
| *HTRA1* | promoter | | 57997 | 710594798 | C/T | | Noncoding |
| *HTRA*1 | promoter | | 57992 | 710594798 | C/T | | Noncoding |
| *HTRA1* | promoter | rs2672598 | 57982 | 710594798 | TIC | | Noncoding |
| *HTRA*1 | intron 1 | | 57018 | 710648330 | C/A | | Noncoding |
| *HTRA*1 | intron 1 | | 56970 | 710648330 | C/T | | Noncoding |
| *HTRA1* | intron 2 | | 30047 | 27864 | C/T | | Noncoding |
| *HTRA1* | intron 2 | | 29931 | 27864 | A/G | | Noncoding |
| *HTRA1* | intron 3 | rs2239586 | 29429 | 27866 | C/T | | Noncoding |
| *HTRA1* | intron 3 | rs2239587 | 29355 | 27868 | G/A | | Noncoding |
| *HTRA1* | exon 4 | | 12401 | 27868 | C/T | | Synonomous |
| *HTRA1* | intron 4 | rs2672582 | 12164 | 27870 | C/T | | Noncoding |
| *HTRA1* | intron 5 | | 11659 | 27870 | GTTT/- | | Noncoding |
| *HTRA1* | intron 5 | rs2672583 | 11578 | 27870 | C/T | | Noncoding |
| *HTRA1* | intron 5 | | 11577 | 27871 | A/G | | Noncoding |
| *HTRA1* | intron 5 | | 10679 | 27871 | C/T | | Noncoding |
| *HTRA1* | intron 6 | rs2672585 | 10267 | 27871 | G/A | | Noncoding |
| *HTRA1* | intron 6 | | 10263 | 27873 | C/G | | Noncoding |
| *HTRA1* | intron 7 | | 8846 | 27875 | C/G | | Noncoding |
| *HTRA1* | Intron 7 | | 7394 | 27875 | * | | Noncoding |
| *HTRA1* | Intron 7 | | 7385 | 27875 | † | | Noncoding |
| *HTRA1* | Intron 7 | | 7393 | 27875 | A/T | Noncoding | |
| *HTRA1* | exon8 | rs11538140 | 7136 | 27875 | C/T | Synonymous | |
| *HTRA1* | intron8 | rs2272599 | 7069 | 27875 | G/A | Noncoding | |
| *HTRA1* | intron 8 | rs2293871 | 4993 | 27877 | T/C | Noncoding | |
| *HTRA1* | exon 9 | | 4744 | 27877 | C/T | Synonomous | |

| | | | | | | | |
|---|---|---|---|---|---|---|---|
| * TAAATAAAA/- (SEQ ID No. 1) † ATAAAAAAAATAAAT/- (SEQ ID No. 2) | | | | | | | |

The primer pair (excluding the M13 tail) used for the sequencing of each fragment is given in Table 7.

**Table 7. Forward and reverse primers used for regions resequenced in the PLEKHA1 and HTRA1 genes**

| Gene | Region | Forward primer | Reverse primer |
|---|---|---|---|
| *HTRA1* | promoter | CGGATGCACCAAAGATTCTCC (SEQ ID No.3) | TTCGCGTCCTTCAAACTAATGG (SEQ ID No.4) |
| *HTRA1* | exon1 | AGCCGGAGCACTGCGAGGG (SEQ ID No.5) | CGCGAAGCTCGGTTCCGAGG (SEQ ID No.6) |
| *HTRA1* | exon 2 | ACGTTTTTGTGGTGAACCTGAGC (SEQ ID No.7) | GCAACAGCCACACACACCTAGC (SEQ ID No.8) |
| *HTRA1* | exon 3 | GCCCGATATATAAAGGAGCGATGG (SEQ ID No.9) | AGAAGTTTTCCTGAGCCCCTTCC (SEQ ID No.10) |
| *HTRA1* | exon 4 | GGGATGTTAGTTGTGAGCTCAGTTCC (SEQ ID No.11) | GCACTAGAATCCACATGGCTTGG (SEQ ID No.12) |
| *HTRA1* | exon 5 | CTGGGCTTCAGAGAGAAAATCTCC (SEQ ID No.13) | ATCCGTAGGGTCATTTGCAAGC (SEQ ID No.14) |
| *HTRA1* | exon 6 | AGTGCCGACCTGGAGTATGTGC (SEQ ID No.15) | GGTGAAATGTCTGTGACCTTCTGC (SEQ ID No.16) |
| *HTRA1* | exon 7 | GTACCCTTCTGTGGCCCTTCC (SEQ ID No.17) | AAGGGGCCAAGGCTAATGACC (SEQ ID No.18) |
| *HTRA1* | exon 8 | CAGTGAACTGAGATCGTACCACTGC (SEQ ID No.19) | AGACAGAAGGCACCCTCCTATGG (SEQ ID No.20) |
| *HTRA1* | exon 9 | CGTGCCTGACCCACTGATGG (SEQ ID No.21) | CCCAAGCTGGCAAGAAAAAGC (SEQ ID No.22) .. |
| *PLEKHA1* | exon 2 | ACCTTACCTAATGTTGGCAAG (SEQ ID No.23) | GAAGACAAATCTAAAGCCTGTATAG (SEQ ID No.24) |
| *PLEKHA1* | exon 3 | TATTTCCCCCTTGCTTTCAGG (SEQ ID No.25) | CCTAAACGTAGTAATCAGGTACC (SEQ ID No.26) |
| *PLEKHA1* | exon 4 | CTCTTACAGTTGGGAACTGCATCC (SEQ ID No.27) | GGGGGTGCAAAATGTTATTTCC (SEQ ID No.28) |
| *PLEKHA1* | exon 5 | AGAAATGCTAGCCAAGTGTGG (SEQ ID No.29) | GCTTGAGTATGAAACCTGTTGG (SEQ ID No.30) |
| *PLEKHA1* | exon 6 | GAACTAGTACCTGCCCGAGTAAGC (SEQ ID No.31) | GGTGAAAAGTACATGAAGAAAGGC (SEQ ID No.32) |
| *PLEKHA1* | exon 7 | CAGGACTTGTGCAAAACAAGAGG (SEQ ID No.33) | CCCCTATTTTATCTCCTGACTCTCC (SEQ ID No.34) |
| *PLEKHA1* | exon 8 | CTGGGTAGCTAGAGAGGGATGAGG (SEQ ID No.35) | GTGGAATGCTGCTTTGAAGATAGG (SEQ ID No.36) |
| *PLEKHA1* | exon 9 | TGTGCTGGATGGTTTAAGAAGG (SEQ ID No.37) | TGTCAAATCTGATGGCCTAACC (SEQ ID No.38) |
| *PLEKHA1* | exon 10 | TGGGTTTGCTAAATCAGTGC (SEQ ID No.39) | CCCACTTCCTGAACATATAACC (SEQ ID No.40) |
| *PLEKHA1* | exon 11 | CATTATTGACGCCTGTTGATGG (SEQ ID No.41) | CTTACATGATCCTGATCACACACC (SEQ ID No.42) |
| *PLEKHA1* | exon 12 | TGCACATTTATGCTGCATGG (SEQ ID No.43) | CAGAGCTTGTTCAGTCACTTTGG (SEQ ID No.44) |
| *PLEKHA1* | exon 12 | CCTCTCGCAGCAACTCTTTGG (SEQ ID No.45) | CCCGAATGAGAACACACAATGC (SEQ ID No.46) |

### Additional Genotyping

Following the identification of rs11200638, all 270 case and control samples were genotyped for rs11200638 using the custom TaqMan SNP genotyping assay (Applied Biosystems). Genotypes were obtained for 97 cases and 126 controls.

### Mouse Real-time PCR

Whole retinas were isolated from C57/Black6 mice aged post-natal 1day, 7days, 1 month, 3 months, 6 months, 9 months, and 16 months as well as from 3 month old Rdl mice. Total RNA was extracted by TRIzol (Invitrogen). Total RNA (2ug) was reverse transcribed to cDNA using the SuperTranscript kit (Invitrogen).

The primer pair for HTRA1 was 5'-TGGGATCCGAATGATGTCGCT (Forward) (SEQ ID NO. 47) and 5'-ACAACCATGTTCAGGGTG (Reverse) (SEQ ID NO. 48) with a length of 237bp. The annealing temperature was 58°C. The Syber Green reagent (Bio-Rad) was employed for the PCR product labeling and the iCycler (Bio-Rad) was used for performing PCR and data collection. Semiquantative PCR was done at a total reaction volume of 25ul, including 2.5ul of 10x High Fidelity PCR buffer (Invitrogen), 1.5ul of MgS04 (50mM, Invitrogen), 0.4 ul of dNTP (25mM, Invitrogen), 0.2ul of Taq DNA Polymerase High Fidelity (Invitrogen), 0.2ul of primers (0.1mM), and 0.2ul of cDNA.

### Computational analysis of the HERA1 promoter

The promoter sequences for the human and mouse HTRA1 genes were obtained from the UCSC Genome Bioinformatics website (www.genome.ucsc.edu). The possible transcription factor (TF) binding sites were examined in the -2,000 to +100 bp region of each promoter sequence using the positional weighting matrices extracted from the TRANSFAC databases (www.gene-regulation.com/pub/databases.html). The footprints of sequence conservation between human and mouse promoters were generated using the DnaBlockAligner program from the Wise 2.0 software package (www.ebi.ac.uk/Wise2/). Within the mouse promoter sequence, -407 was identified as the -512 G→A SNP site in humans. Only those TF binding sites that covered the SNP and were located in the human and mouse conserved promoter region were considered suitable. Results of the computational analysis are shown in Figure 3.

Chromatin immunoprecipitation (ChIP) 1x10⁸ HeLaS3 cells were treated with formaldehyde (final concentration of 1%) for 10 min to crosslink proteins to their DNA binding targets and quenched with glycine in phosphate buffered saline (PBS) at a final concentration of 125 mM. Cells were washed twice with cold 1x PBS. The nuclear extract was prepared by swelling the cells on ice for 15 min in a hypotonic buffer (20 mM Hepes, pH 7.9, 10 mM KCl, 1 mM EDTA, pH 8, 10% glycerol, 1 mM DTT, 0.5 mM PMSF, 0.1 mM sodium orthovanadate, and protease inhibitors), followed by dounce homogenization (30 strokes). The nuclei were pelleted by brief centrifugation and lysed in radioimmunoprecipitation (RIPA) buffer (10 mM Tris-Cl, pH 8.0, 140 mM NaCl, 0.025% sodium azide, 1% Triton X-100, 0.1% SDS, 1% deoxycholic acid, 0.5 mM PMSF, 1 mM DTT, 0.1 mM sodium orthovanadate, and protease inhibitors) for 30 min on ice with repeated vortexing. The extract was sonicated with a Branson 250 Sonifier to shear the DNA (Output 20%, 100% duty cycle, five 30 second pulses) and the samples were clarified by centrifugation at 14,000rpm at 4°C for 15 min. 200 µL of extract was set aside for the purification of a control input DNA sample. AP-2α or SRF-DNA complexes were immunoprecipitated from the sonicated extract with an anti-AP-2α (C-18) or anti-SRF (H-300) antibody (Santa Cruz Biotechnology) overnight at 4°C with gentle agitation. Control chromatin IP DNA was prepared using normal polyclonal rabbit IgG (Santa Cruz Biotechnology). Each immunoprecipitation sample was incubated with protein A-agarose (Upstate Biotechnology) for 1 hour at 4°C followed by three washes with RIPA buffer and one wash with 1x PBS. The antibody-protein-DNA complexes were eluted from the beads by addition of 1% SDS, 1x TE (10 mM Tris-Cl, pH 7.6, 1 mM EDTA, pH 8) and incubation at 65°C for 10 min, followed by a second round of elution with 0.67% SDS in 1x TE, incubation for another 10 min at 65°C, and then gentle vortexing for 10 min. The beads were removed by centrifugation and the supernatants were incubated at 65°C overnight to reverse the crosslinks. To purify the DNA, as well as the input DNA sample, RNaseA was added (200 µg/sample, in 1x TE) and then the samples were incubated at 37°C for 2 hours, followed by an incubation with proteinase K solution (400 µg/ml proteinase K) for 2 hours at 45°C. Lastly, a phenol:chloroform:isoamyl alcohol extraction was performed and the DNA was recovered by ethanol precipitation. A more detailed description of the procedure can be found in (S. E. Hartman et al., Genes Dev 19, 2953 (2005)).

### Quantitative PCR analysis of ChIP DNA samples

The normal rabbit IgG, AP-2α, and SRF ChIP DNA samples were analyzed by quantitative PCR in order to test for enrichment of specific binding sites. Primers were designed to flank the candidate target region upstream of the HTRA1 gene: (-574 to -331; Forward: 5'- TCACTTCACTGTGGGTCTGG-3' (SEQ ID No. 49); Reverse: 5'-GGGGAAAGTTCCTGCAAATC -3') (SEQ ID No. 50). Primers were also designed to flank known AP-2α and SRF-bound human promoter regions to serve as positive controls for the ChIP PCR tests (S. Decary et al., Mol Cell Biol 22, 7877 (2002)). For AP-2α, regions upstream of insulin-like growth factor binding protein 5 *(IGFBP-5;* -94 to +73; Forward: 5'-CTGAGTTGGGTGTTGGGAAG-3' (SEQ ID No. 51); Reverse: 5'-AAAGGGAAAAAGCCCACACT-3') (SEQ ID No. 52) and E-cadherin (*ECAD*; - 174 to -7; Forward: 5'-TAGAGGGTCACCGCGTCTATG-3' (SEQ ID No. 53); Reverse: 5'-GGGTGCGTGGCTGCAGCCAGG -3') (SEQ ID No. 54) were chosen (K. P. Magnusson et al., PLoS Med 3, e5 (2006)). The positive controls for SRF were upstream of Fos-related antigen 1 *(FRA-1;* -238 to -91; Forward: 5'-GCGGAGCTCGCAGAAACGGAGG-3' (SEQ ID No. 55); Reverse: 5'-GGCGCTAGCCCCCTG ACGTAGCTGCCCAT-3') (SEQ ID No. 56) (P. Adiseshaiah, S. Peddakama, Q. Zhang, D. V. Kalvakolanu, S. P. Reddy, Oncogene 24,4193 (2005)) and early growth response protein *(EGR-1;* -196 to -30; Forward: 5'-CTAGGGTGCAGGATGGAGGT-3' (SEQ ID No. 57); Reverse: 5'-GCCTCTATTTGAAGGGTCTGG-3') (SEQ ID No. 58) (U. Philippar et al., Mol Cell 16, 867 (2004)). A negative control human promoter region, B-lymphoma and BAL-associated protein *(BBAP),* was also tested (-151 to +59; Forward 5'-CAGACAGCACAGGGAGGAG-3' (SEQ ID No. 59); Reverse 5'-ACTTGTACACCCGCACGAG-3') (SEQ ID No. 60). Quantitative PCR reactions were performed using an ABI Prism 7000 Sequence Detection System and SYBR Green Master Mix (MJ Research) and 5% DMSO. Cycling conditions were as follows: 95°C for 5 min, 40 cycles of 95°C for 30 sec, 52°C for 30 sec, 72°C for 30 sec, a final extension period of 72°C for 10 min, followed by a 60-95°C dissociation protocol. The ΔΔCt and fold change values were calculated relative to reference PCR reactions.

### Reporter Assay

The effect of the SNP rs11200638 on the activity of the HTRA1 promoter was assessed using a luciferase assay on transfected ARPE19 (immortalized human retinal pigment epithelium) cells from the 32nd passage and HeLaS3 cells. Constructs were designed according to the scheme in Table 8 with one contruct containing the rs11200638 wild-type allele, another containing the mutant allele and a third with no insert. Cells were grown in high glucose Dulbecco's Modified Eagle's Medium (DMEM) + 10% Fetal Bovine Serum (FBS) + 0.1% Gentamicin. For transfection the medium was changed to reduced serum artificial medium (Opti-MEM I) without Gentamicin. Cultures were transfected when they reached 80% confluence for the HeLaS3 cells and 50% confluence for the ARPE19 cells. Transfection was carried out with lipofectamine 2000 (2ul/ml; Invitrogen), enhanced Green Fluorescence Protein (eGFP; 1.2ug/ml) and the constructs (0.7ug/ml, 1.4ug/ml or 2.1ug/ml for HeLaS3 and 1.4ug/ml for ARPE19). As an additional control, each cell type was transfected with lipofectamine alone (Control). Cells were incubated with the transfection reagents for 8 hours. After removal of the transfection reagents, cells were allowed to grow for an additional 48 hours before the luciferase assay was performed. 150ul of the lysis buffer was added to each well. 100ul of the resulting lysate was loaded onto a black 96-well plate and 100ul of the luciferase substrate (Bright-Glo; Promega) was added to each well. Data collection (both GFP fluorescence intensity and luciferase activity) was performed on a Packard Fusion 96-well plate reader. Independent experiments were repeated three times for each construct dose and construct type for the HeLaS3 cells and six times for each construct type for the ARPE19 cells.

**Table 8. Experimental design of the HTRA1 promoter reporter assay. Information on the reporter constructs used in the assay.**

| Construct name | Vector | Insert | SNP genotype |
|---|---|---|---|
| *HTRA1*-AA | PGL2-Basic | -834 to +119 | mutant |
| *HTRA1*-GG | PGL2-Basic | -834 to +119 | wild-type |
| Blank vector | PGL2-Basic | None | N/A |
| eGFP | GFP reporter vector for transfection efficiency control | | |

### EXAMPLE 1

To identify novel genetic variant(s) that predispose individuals to the wet, neovascular AMD phenotype in patients of Asian descent Applicants identified 96 patients previously diagnosed with wet AMD and 130 age matched control individuals who were AMD-free (L. Baum et al., Ophthalmologica 217, 111 (2003), C. P. Pang et al., Ophthalmologica 214, 289 (2000)) from a cohort of Southeast Asians in Hong Kong. Epidemiological observations indicate that neovascular AMD is more prevalent among Asians than Caucasians (A. C. Bird, Eye 17, 457 (2003), R. Klein et al., Ophthalmology 113, 373 (2006), T. S. Chang, D. Hay, P. Courtright, Can J Ophthalmol 34, 266 (1999)), and the soft indistinct drusen that are characteristic of dry AMD are rarely seen in Asian individuals (M. A. Sandberg, A. Weiner, S. Miller, A. R. Gaudio, Ophthalmology 105, 441 (1998), M. Uyama et al., Br J Ophthalmol 84, 1018 (2000), M. Yuzawa, K. Hagita, T. Egawa, H. Minato, M. Matsui, Jpn J Ophthalmol 35, 87 (1991)). The CFH Y402H variant that occurs frequently in Caucasians (>35%) has been shown to occur less frequently in individuals of Japanese and Chinese ancestry (<5%) (N. Gotoh et al., Hum Genet 120, 139 (2006), H. Okamoto et al., Mol Vis 12, 156 (2006),M. A. Grassi et al., Hum Mutat 27, 921 (2006)). Retinal fundus photographs were examined from each of the 226 study participants. Indocyanine Green Dye (ICG) angiography was performed to exclude cases with polypoidal choroidal vasculopathy (PCV) and to verify that CNV (AMD grade 5) was present in all cases. The AMD cases and controls had a mean age of 74. Other characteristics of the study population are summarized in Table 1.

Applicant conducted a whole-genome association study on this Asian cohort to scan for single nucleotide polymorphisms (SNPs) using previously described genotyping and data quality surveillance procedures (C. P. Pang et al., Ophthalmologica 214, 289 (2000)). Of the 97,824 autosomal SNPs that were informative and passed the quality control checks, rs10490924 was the only polymorphism that showed a significant association with AMD using the Bonferroni criteria (Table 9). The allele frequency chi-square test yielded a P-value of 4.1 × 10⁻¹² (Table 9). The OR was 11.1 (95% confidence interval [CI] 4.83-25.69) for those carrying two copies of the risk allele when compared to wild-type homozygotes, but was indistinguishable from unity, 1.7 (95% CI 0.75-3.68), for those having a single risk allele. The risk homozygote accounted for 86% of the population attributable risk (PAR), although this number may be artificially inflated since the risk allele was carried by more than half (∼55%) of the AMD cohort (Table 9). When likelihood ratio tests were adjusted for gender and smoking status or when genomic control methods were applied to control for population stratification, there was little change in significance levels.

**Table 9. Association, odds ratios and population attributable risk (PAR) for AMD in a Chinese population. Odds ratio and PAR compare the likelihood of AMD in individuals with the listed genotype of risk allele versus those homozygous for the wild-type allele.**

| SNP (alleles) | Risk Allele | Allelic *χ*² nominal P | Odds ratio* (95% Cl) | PAR* (95%Cl) | Odds ratio† (95%Cl) | PAR†(95% Cl) |
|---|---|---|---|---|---|---|
| | | | | | | |
| rs10490924(G/T) | T | 4.08 × 10⁻¹² | 1.66 | 29% | 11.14 | 86% |
| | | | (0.75-3.68) | (0-63%) | (4.83-25.69) | (69%-94%) |
| rs11200638(G/A) | A | 8.24 × 10⁻¹² | 1.60 | 27% | 10.0 | 84% |
| | | | (0.71-3.61) | (0-61%) | (4.38-22.82) | (66%-93%) |

| | | | | | | |
|---|---|---|---|---|---|---|
| *Heterozygous risk individuals compared to the wild-type homozygotes. †Homozygous risk individuals compared to the wild-type homozygotes. | | | | | | |

SNP rs 10490924 resides between two genes on chromosome 10q26 (Figure 1): *PLEKHA1* encoding a pleckstrin homology domain-containing protein (GenBank ID 59338) and HTRA1 encoding a heat shock serine protease also known as *PRSS11* (GenBank ID 5654). The low sequence homology across species in the intergenic region containing rs10490924 indicates that it is not evolutionarily conserved (Figure 1). Chromosome 10q26 has been linked to AMD in many independent family studies and this linkage region was previously narrowed to SNP rs 10490924 (P. Armitage, G. Berry, Statistical Methods in Medical Research (Balckwell Scientific Publications, 1971)). SNP rs10490924 was originally thought to result in a protein coding change in the hypothetical locus LOC387715 (A. Rivera et al., Hum Mol Genet 14, 3227 (2005), J. Jakobsdottir et al., Am J Hum Genet 77, 389 (2005)). Based on evidence of only a single cDNA sequence found in placental tissue, LOC387715 was subsequently removed from the GenBank database. Applicants hypothesized that SNP rs1 0490924 might be a surrogate marker that is correlated, or is in linkage disequilibrium (LD), with the putative AMD disease-causing variant in the vicinity. Haplotype analyses using Applicant's genotype data or data from the International HapMap Project were unsuccessful in identifying where the functional site resides.

Applicant therefore sequenced the entire local genomic region, including promoters, exons and intron-exon junctions of both PLEKHA1 and HTRA1, in search of the functional variant. Based on the genotypes of the marker SNP rs10490924, 50 cases that were homozygous for the risk allele and 38 controls that were homozygous for the wild-type allele were sequenced. Of the 43 SNPs or insertion/deletion polymorphisms identified (Figure 1 and Table 6), one SNP (rs11200638), located 512 base pairs (bp) upstream of the HTRA1 putative transcriptional start site and 6,096 bp downstream of SNP rs10490924, exhibited a complete LD pattern with SNP rs10490924. Genotyping of the entire cohort revealed that SNP rs11200638 occurred at frequencies similar to those for SNP rs10490924 (*P* = 8.2×10⁻¹² for the allele association χ² test), and the two SNPs were almost in complete LD (D'> 0.99) Table 9.

### EXAMPLE 2

The SNP rs11200638 is located 512 base pairs (bp) upstream of the transcription start site of the HTRA1 gene (also known as *PRSS11, NM 002775).*

Computational analysis of the HTRA1 promoter sequence predicted that SNP rs11200638 resides within putative binding sites for the transcription factors adaptor-related protein complex 2 alpha (AP2α and serum response factor (SRF). This DNA segment, containing the wild-type allele, is part of a CpG island and closely matches the consensus response sequences of these two transcription factors (Figure 3). The presence of the risk allele was predicted to alter the affinity of AP2α and SRF for the HTRA 1 promoter. In addition, promoter analysis with MatInspector (Genomatix Software GmbH) suggested that the sequence variation at SNP rs11200638 might alter the binding of the Sp (Specific protein) transcription factor family member.

To verify that the predicted transcription factors bind to the HTRA1 promoter in cultured human cells, Applicant performed chromatin immunoprecipitation (ChIP) followed by quantitative real-time PCR analyses. Lysates were prepared from growing human cervical carcinoma cells (HeLaS3) heterozygous at rs11200638 and ChIP was conducted using rabbit polyclonal antibodies against AP2α or SRF. Quantitative PCR tests of the ChIP DNA samples confirmed that both AP2α and SRF bind upstream of the HTRA1 gene (Figures 2 and 3).

To investigate the influence of SNP rs11200638 on the HTRA1 promoter, human ARPE19 (retinal pigment epithelium) and HeLaS3 cells were transiently transfected with a luciferase reporter plasmid driven by the HTRA1 promoter harboring either the wild-type (GG) or the risk homozygote (AA) genotype. Preliminary results showed a persistent trend of higher luciferase expressions with the AA compared to the GG genotype.

The Following Methods and Materials were used in the work described herein, particularly Examples 3, 4, and 5.

### Patients

This study was approved by the University of Utah Institutional Review Board. All subjects provided informed consent prior to participation in the study. AMD patients were recruited at the Moran Eye Center (University of Utah), as were normal age-matched controls (individuals age 60 years or older with no drusen or RPE changes). All participants went through a standard examination protocol and visual acuity measurements. Slitlamp biomicroscopy of the fundi using a 90 diopter lens were performed. A pair of stereoscopic color fundus photographs (50°) were taken, centered on the fovea using a Topcon fundus camera (Topcon TRV-50VT, Topcon Optical Company, Tokyo, Japan) by trained ophthalmic photographers. Grading was carried out according to the standard grid classification system suggested by the International ARM Epidemiological Study Group for agerelated maculopathy (ARM) and AMD (A. C. Bird et al., Surv Ophthalmol 39, 367 (1995)). All abnormalities in the macula were characterized according to 1) type, size, and number of drusen, 2) RPE hyperpigmentation or hypopigmentation, and 3) advanced AMD stages including geographic atrophy (GA, dry AMD), and choroidal neovascularization (CNV, wet AMD). A total of 581 AMD patients (392 wet AMD, 189 soft confluent drusen) and 309 age and ethnicity matched normal controls participated in this study (Table 10).

**Table 10. Characteristics of AMD Cases and Controls Matched for Age and Ethnicity**

| | Cases | Controls |
|---|---|---|
| Mean Age | 77 | 72 |
| Gender (M/F) | 291/290 | 104/205 |
| AMD (total) | 581 | 309 |
| AMD (wet) | 392 | |
| AMD (soft confluent drusen) | 189 | |

### Genotyping

The initial Utah cohort of 442 Caucasion AMD patients, including 265 wet AMD and 177 soft confluent drusen, was genotyped and allele frequencies were compared to 309 age and ethnicity matched normal controls. The expanded sample for second stage genotyping of rs10490924 and rs11200638 included 581 AMD patients (392 wet AMD, 189 soft confluent drusen).

For the rs11200638 genotype, Applicant PCR-amplified genomic DNA extracted from AMD and control patient blood samples. Oligonucleotide primers, forward 5'-ATGCCACCCACAACAACTTT-3' (SEQ ID. No. 61) and reverse, 5'-CGCGTCCTTCAAACTAATGG-3' (SEQ ID. No. 62) were used in PCR reactions containing 5% DMSO. DNA was denatured at 95°C-3 minutes, followed by 35 cycles, 94°C-30 seconds, 52°C-30 seconds, and 72°C-45 seconds per cycle. The PCR product was digested with Eag I to identify the G allele. For rs10490924, forward primer 5'-TACCCAGGACCGATGGTAAC-3' (SEQ ID. No. 63) and reverse primer 5'GAGGAAGGCTGAATTGCCTA-3' (SEQ ID. No. 64) were used for PCR amplification, PVUII digestion was used to identify the G allele. The remaining 13 SNPs were genotyped using the SNaPshot method on an ABI 3130 genetic analyzer (Applied Biosystems, Foster City, CA) according to the manufacturer's instructions. CFH genotyping was performed according to published methods (K.P. Magnusson et al.,. PLoS Med 3, e5 (Jan, 2006)).

### Data analysis

### 10g26

The chi-squared test for trend for the additive model over alleles was performed to assess evidence for association. Odds ratios and 95% confidence intervals were also calculated to estimate risk size for the heterozygotes and homozygotes for the risk alleles.

### Two-locus analyses (CFHY402H at 1g31 and rs11200638 at 10g26)

Two-locus analyses were performed for the CFH rs1061170 (Y402H) variant at 1 q31 and rs1120063 8 for 10g26. A contingency table based on case-control status and two-locus genotype combination was constructed. The two-locus genotype combinations across CFHY402H and rs11200638 were TT/GG, TT/AG, TT/AA, CT/GG, CT/AG, CT/AA, CC/GG, CC/AG, and CC/AA. This global, two-locus 9x2 contingency table was tested with a chi-squared statistic on 8 degrees of freedom. Odds ratios and 95% confidence intervals, comparing each genotypic combination to the baseline of homozygosity for the common allele at both loci (TT/GG), was calculated. For the risk genotypes identified, Applicant calculated population attributable risks (PAR) which indicates the proportion of total disease risk attributable to the risk genotypes, using the Levin formula (M. L. Levin, Acta Unio Int Contra Cancrum 9, 531 (1953)).

### HTRA1 immunohistochemistry

AMD donor eyes were obtained from Utah Lions Eye Bank. Cryosections from paraformaldehydefixed eyes were incubated in 0.3% H2O2 in methanol to quench endogenous peroxidase activity. Immunohistochemistry was performed using 5µg/ml monospecific anti-human HTRA1 polyclonal antibody (J. Chien et al., J Clin Invest 116, 1994 (Jul, 2006)). The VectorStain Elite ABC kit (Vector Laboratories, Burlingame, CA) and the VIP peroxidase substrate (Vector Laboratories) were used for HTRA1 detection and immunolabeling was captured using Nomarski optics on a Nikon Eclipse 80i microscope.

### Semiquantitative RT-PCR of HTRA1 mRNA in human lymphocyte samples

A commercial real-time PCR system (Opticon; MJ Research, Watertown, MA) was used for quantifying *HTRA1* transcript levels from patient blood lymphocyte samples. Total RNA was extracted (RNeasy; Qiagen, Valencia, CA) from peripheral lymphocytes of blood samples and reversetranscription PCR (RT-PCR) was performed using the QuantiTect SYBR Green RT-PCR kit (Qiagen). *HTRA I* primers (forward primer 5'-AGCCAAAATCAAGGATGTGG-3' (exon 3) (SEQ ID NO. 65) and reverse primer 5'-GATGGCGACCACGAACTC-3' (exon 4)) (SEQ ID NO. 66) and 100 nanograms (ng) total RNA from each sample were used for one step RT-PCR reactions. Standard curves were generated from 0 to 400 ng total RNA from patient lymphocyte samples. A house-keeping gene Glyseraldehyde-3-phosphate dehydrogenase (GAPDH) was amplified in parallel reactions (forward primer 5'- CTGCACCACCAACTGCTTAG 3' (exon 7) (SEQ ID NO. 67) and reverse primer 5'- GTCTTCTGGGTGGCAGTGAT -3') (SEQ ID NO. 68) and used to normalize HTRA1 values. HTRA1 and GAPDH standard curves showed similar amplification kinetics. Three to four patients for GG and AA genotypes were assayed in duplicate reactions and duplicate runs. Results are presented as percent increase in HTRA1 RNA levels for AA samples relative to GG samples (Figure 4B).

### Western analysis of HTRA1 expression in human RPE

25 µg of total protein of retinal pigment epithelium from four wet AMD eyes with an AA genotype and six normal eyes with a GG genotype was subj ect to SDS-PAGE. Western blotting was performed using 1.5 µg/ml anti-human HtrA1 polyclonal antibody (J. Chen et al., J Clin Invest 116, 1994 (Jul, 2006)). HTRA1 protein expression level was normalized to (3-actin. Statistical significance was examined using an independent samples t-test (SPSS version 13.0).

### EXAMPLE 3

To identify the critical gene at the chromosome locus 10q26 in a Caucasian cohort in Utah, applicant genotyped 442 AMD cases and 309 controls, using a panel of 15 single-nucleotide polymorphisms (SNPs) centered around the highest risk associated SNP, rs 10490924 was found to have a significant association signal [*P* =8.1 x 10⁻⁸ for an additive allele-dosage model, ORₕₑₜ = 1.35 (0.99, 1.86), ORₕₒₘ = 6.09 (3.27, 11.34), T allele: 39.7% in cases versus 24.7% in controls]. However, of the 15 SNPs analyzed, rs11200638 was the most significantly associated variant [*P* = 1x 10⁻⁹, ORₕₑₜ = 1.86 (1.35,2.56), ORₕₒₘ = 6.56 (3.23, 13.31), A allele: 40.3% in cases versus 25.2% in controls] (Figure 4A and Table 11).

**Table 11. Association Results for 15 SNPs at 10g26 in AMD Cases and Controls.**

| SNP | by | chi-trend | trend_p | ORhet | ORhom | chi_dom | dom_p | ORdom |
|---|---|---|---|---|---|---|---|---|
| rs986960 | 12408254 | 0.5141005 | 0.4733694 | 0.91(0.64,1.3 | 0.85 | 0.4099402 | 0.522 | 0.89 (0.64, 1.26) |
| rs1998345 | 12411429 | 8.1847003 | 0.0042245 | 1.37(0.99.1.9 | 2.02 | 6.1691034 | 0.013 | 1.48 (1.09,2.02) |
| rs2901307 | 12411843 | 5. 0544893 | 0.0245621 | 0.79(0.55,1.1 | 0.59 | 3.0248076 | 0.082 | 0.73 (0.52,1.04) |
| rs4146894 | 12414537 | 16.549416 | 4.74E-05 | 1.76(1.22.2.5 | 2.32 | 15.445303 | 8.49E-05 | 1.96 (1.40,2.74) |
| rs2421016 | 12415750 | 11.094448 | 0.0008661 | 1.82(1.24,2,6 | 2.21 | 11.989527 | 0.0005352 | 1.91 (1.32,2.77) |
| rs1045216 | 12417918 | 0 | 1 | 0.88(0.64,1.2 | 1.12 | 0.2458672 | 0.62 | 0.92 (0.67,1.26) |
| rs1049092 | 12420443 | 32 1381 | 8 14E-08 | 1.35(0.99,1.8 | 6.09(3.27,11. | 15.372106 | 8.80E-05 | 1.81 (1.35,2.45) |
| rs3750847 | 12420541 | 27.1378 | 1.86E-07 | 1.44(1.04.1.9 | 5.99(2.98,12. | 14.608167 | 0.0001324 | 1.82 (1.34,2.47) |
| rs3750846 | 12420555 | 18.646568 | 1.57E-05 | 1.9 | 4.86(2.32,10. | 10.332306 | 0.0013074 | 1.65 (1. 22,2. 24) |
| rs2014307 | 12420762 | 24.366379 | 5.90E-07 | 0.61(0.44 .0.8 | 0.23 | 14.867718 | 0.0001154 | 0.54 (0.39,0.74) |
| rs1120063 | 12421052 | 37.2931 | 1.02E-09 | 1.86(1.35,2.5 | 6.56(3.23,13. | 28.22876 | 3.82E-07 | 2.21 (1.62,3.01) |
| rs1049331 | 12421126 | 0.8081998 | 0.368653 | 0.71(0.49,1.0) | 0.88 | 2.4978763 | 0.114 | 0.75 (0.52,1.07) |
| rs4752700 | 12422760 | 1.4022982 | 0.236339 | 0.65(0.45,0.9 | 0.85 | 4.176009 | 0.041 | 0.69 (0.49,0.99) |
| rs2300431 | 12423280 | 1.6662006 | 0.1967684 | 1.0 | 0.81 | 2.4436668 | 0.118 | 0.76 (0.53, 1.07) |
| rs714816 | 12424633 | 6,812544 | 0.0090517 | 1.11(0.80,1.5 | 2.26 | 2.2571312 | 0.133 | 1.27 (0.93,1.73) |

In terms of the significance of the association, the TA haplotype across rs10490924 and rs11200638 was superior to rs10490924 (*P* = 2.2 x 10⁻⁹), but inferior to rs11200638. Applicant genotyped an additional 139 AMD patients for these two variants. The results for both SNPs increased in significance (rs 10490924, *P* =^{1.2}× 10⁻⁸; rs1 1200638, *P* =1.6 x 10⁻¹¹), with variant rs11200638 remaining the best single variant explaining the association [ORₕₑₜ =1.90 (1.40, 2.58), ORₕₒₘ = 7.51 (3.75, 15.04)].

### EXAMPLE 4

Complement factor H (CFH) has been suggested to mediate drusen formation (G. S. Hageman et al., Proc Natl Acad Sci USA 102, 7227 (2005)). In Applicant's previous whole-genome association study in which the presence of large drusen was the primary phenotype under investigation, the CFH Y402H variant was shown to be a major genetic risk factor (R. J. Klein et al., Science 308, 385 (2005)). More recently, it has been reported that the highest odds ratio (OR) for CFH Y402H was seen for cases with AMD grade 4 (i.e., the presence of CGA) in comparison to AMD grade 1 controls (E. A. Postel et al., Ophthalmology 113, 1504 (2006)). An association between AMD and CFH Y402H, as well as other intronic CFH variants, has been demonstrated for more than ten different Caucasian populations (J. Maller et al., Nat Genet 38, 1055 (2006), S. Haddad, C. A. Chen, S. L. Santangelo, J. M. Seddon, Surv Ophthalmol 51, 316 (2006), M. Li et al., Nat Genet 38, 1049 (2006), A. Thakkinstian et al., Hum Mol Genet 15, 2784 (2006)). Applicant conducted association analyses based on genotypes at both rs11200638 and the CFH rs1061170 (Y402H) variant at chromosome 1q31. In a global two-locus analysis enumerating all nine two-locus genotype combinations, the association with AMD was significant (x² = 56.56, 8 df; *P* = 2.2 x 10⁻⁹). Table 12 shows the risk estimates for each two-locus genotype combination compared with the baseline ofno risk genotypes (TT at CFHY402H and GG at rs 11200638).

**Table 12. Two-locus odds ratios for HTRA1 rs11200638 and CFH rs1061170. Odds ratios with 95% confidence intervals in parentheses were calculated to compare each genotypic combination to the baseline of homozygosity for the common allele at both loci (TT/GG).**

| SNP *HTRA1* rs11200638 | | | | |
|---|---|---|---|---|
| | | | | |
| *CFH* rs1061170 (Y402H) | | GG | AG | AA |
| | TT | 1.00 | 1.80 (0.93,3.49) | 3.43 (0.62,19.00) |
| | CT | 1.07 (0.59,1.94) | 2.31 (1.28,4.17) | 7.31 (2.68,19.93) |
| | CC | 3.07 (1.50,6.27) | 3.97 (1.93,8.15) | 31.52 (4.01,247.96) |

The association of rs11200638 to AMD was significant when analyzed conditional on the presence of the CFH C risk allele (*P* = 5.9 x 10⁻⁸). In particular, this conditional analysis indicates an allele-dosage effect such that homozygotes for the A risk allele of rs1 1200638 are at an increased risk [ORₕₒₘ = 7.29 (3.18,16.74)] over that of heterozygotes [ORₕₑₜ = 1.83 (1.25, 2.68)] in all AMD cases, even when compared with a baseline that includes individuals who carry the risk genotypes at *CFH.* With an allele-dosage model, the estimated population attributable risk (PAR) for rs11200638 is 49.3%. Consistent with an additive effect, the estimated PAR from a joint model with CFH Y402H (that is, for a risk allele at either locus) is 71.4%.

### EXAMPLE 5

To investigate the functional significance of SNP rs1 1200638 in Caucasians, Applicant used real-time reverse transcription polymerase chain reaction (RT-PCR) to study the expression levels of HTRA1 mRNA in lymphocytes of four AMD patients carrying the risk allele AA and three normal controls carrying the normal allele GG (Figure 4B). The HTRA1 mRNA levels in lymphocytes from AMD patients with the AA genotype were higher by a factor of 2.7 than those in normal controls with the GG genotype (Figure 4B). The mean HTRA1 protein level in RPE of four AMD donor eyes with a homozygous AA risk allele was higher by a factor of 1.7 than that of six normal controls with a homozygous GG allele. The analysis of human eye tissue was limited to four AMD donor eyes with an AA genotype out of the 60 donors for this study. The data suggest a trend toward higher expression with the risk AA allele. Immunohistochemistry experiments revealed that HTRA1 immunolabeling is present in the drusen of three AMD patients.

The HTRA1 gene encodes a member of a family of serine proteases expressed in the mouse retina and RPE (C. Oka et al., Development 131, 1041 (2004)). HTRA1 appears to regulate the degradation of extracellular matrix proteoglycans. This activity is thought to facilitate access of other degradative matrix enzymes, such as collagenases and matrix metalloproteinases, to their substrates (S. Grau et al., J Biol. Chem. 281, 6124 (2006)). Conceivably, overexpression of HTRA1 may alter the integrity of Bruch's membrane, favoring the invasion of choroid capillaries across the extracellular matrix, as occurs in wet AMD. HTRA1 also binds and inhibits transforming growth factor-β (TGF-β), an important regulator of extracellular matrix deposition and angiogenesis (Oka et al., Development 131, 1041 (2004)).

## Claims

1. A method of identifying or aiding in identifying an individual suffering from or at risk for developing age related macular degeneration, of diagnosing or aiding in diagnosing age related macular degeneration in an individual, or of assessing the risk of progression of age related macular degeneration in an individual, comprising:
(I) assaying a sample obtained from the individual for the presence of a nucleotide base other than a G at position 29 in SEQ ID No: 69 of the human HTRA1 gene, wherein the presence of a nucleotide base other than a G at said position of the variant HTRA1 gene indicates that the individual suffers from or is at risk for developing or progression of age related macular degeneration; or
(II)
(a) combining a sample from the individual with a polynucleotide probe that hybridizes, under stringent conditions, to a variation in the non-coding regulatory region upstream of position +1 of the putative transcription start site of the human HTRA1 gene that is correlated with the occurrence of age related macular degeneration in humans, but does not hybridize to a wild-type HTRA1 gene, thereby producing a combination;
(b) maintaining the combination conditions appropriate for hybridization to occur; and
(c) determining whether hybridization occurs, wherein the occurrence of hybridization indicates that the individual is at risk for developing or progression of age related macular degeneration; or
(III)
(a) sequencing a region of the DNA from an individual that comprises the nucleotide at position 29 in SEQ ID No: 69 from the putative transcription start site of the promoter of the HTRA1 gene in humans; and
(b) determining whether a variation of the wild-type sequence of the promoter region of the HTRA1 gene is present in the DNA, wherein the presence of the variation indicates that the individual is at risk for developing or progression of age related macular degeneration;
said variation being a nucleotide base other than a G at position 29 in SEQ ID No: 69.

2. The method of claim 1 wherein said variant corresponds to a single nucleotide polymorphism (G-→A) at position 29 in SEQ ID No: 69 (rs11200638).

3. The method of claim 1 or claim 2, further comprising determining in the sample the presence or absence of an additional variation in a gene that is correlated with the occurrence of age related macular degeneration in humans, wherein the additional variation is other than the variation in the non-coding regulatory region upstream of position +1 of the putative transcription site of a human HTRA1 gene.

4. The method of claim 3 wherein the additional variation is detected using a second probe that hybridizes to the additional variation.

5. The method of claim 3, wherein the additional variation is detected using a second set of primers that hybridize to either side of the additional variation.

6. The method of any one of claims 3 to 5, wherein the additional variation is the presence of histidine at position 402 of the human CFH protein, corresponding to the SNP rs1061170 and/or the presence of an amino acid other than alanine at position 69 of the human protein LOC387715, for example serine, corresponding to SNP rs10490924.

7. The method of any one of claims 1 to 6 wherein the individual is an Asian individual.

8. A diagnostic kit for detecting a variant HTRA1 gene in a sample from an individual, comprising at least one container means and a label and/or instructions for the use of the diagnostic kit in the detection of a variant HTRA1 gene in a sample, wherein said container means has disposed therein:
(a) a polynucleotide probe that hybridizes, under stringent conditions, to a first variation in the non-coding regulatory region upstream of position +1 of the putative transcription site of the human HTRA1 gene that is correlated with the occurrence of age related macular degeneration in humans; or
(b) a polynucleotide primer that hybridizes, under stringent conditions, adjacent to one side of a variation at position 29 in SEQ ID No: 69 of the promoter region of the human HTRA1 gene that is correlated with the occurrence of age related macular degeneration in humans, and optionally a second polynucleotide primer that hybridizes, under stringent conditions, to the other side of the variation at position 29 in SEQ ID No: 69;
wherein the kit further comprises a second probe or pair of primers that detect in the sample the presence or absence of an additional variation that is correlated with the occurrence of age related macular degeneration in humans and wherein said first variation is a nucleotide base other than a G at position 29 in SEQ ID No: 69 and said additional variation is the presence of histidine at position 402 of the human CFH protein, corresponding to the SNP rs 1061170 and/or the presence of an amino acid other than alanine at position 69 of the human protein LOC387715, for example serine, corresponding to SNP rs10490924.

9. A composition for use in a method of treating age related macular degeneration, comprising a pharmaceutically acceptable carrier and a bispecific, single-chain, chimeric, humanized or human antibody that binds to the HTRA1 polypeptide or an antibody specifically reactive with the HTRA1 polypeptide, wherein binding of the antibody to the HTRA1 polypeptide reduces the activity of the HTRA1 polypeptide.

## Patentansprüche

1. Ein Verfahren zum Identifizieren oder zum Unterstützen des Identifizierens eines Individuums, das unter altersbedingter Makuladegeneration leidet oder dem Risiko für die Entwicklung einer altersbedingten Makuladegeneration unterliegt, zum Diagnostizieren oder Unterstützen des Diagnostizierens altersbedingter Makuladegeneration eines Individuums, oder zum Abschätzen des Risikos des Fortschreitens altersbedingter Makuladegeneration eines Individuums, umfassend:
(I) Untersuchen einer Probe, gewonnen von dem Individuum, auf das Vorliegen einer anderen Nukleotidbase als G an Position 29 in SEQ ID No: 69 des humanen HTRA1-Gens, wobei das Vorliegen einer anderen Nukleotidbase als G an besagter Position des abweichenden HTRA1-Gens anzeigt, dass das Individuum unter altersbedingter Makuladegeneration leidet oder dem Risiko für die Entwicklung oder das Fortschreiten einer altersbedingten Makuladegeneration unterliegt; oder
(II)
(a) Kombinieren einer Probe von dem Individuum mit einer Polynukleotidsonde, die unter strengen Bedingungen mit einer Variante in der nicht-kodierenden regulatorischen Region strangaufwärts der Position +1 des putativen Transkriptionsstartpunkts des humanen HTRA1-Gens hybridisiert, die mit dem Auftreten altersbedingter Makuladegeneration bei Menschen korreliert, aber nicht mit einem Wildtyp des HTRA1-Gens hybridisiert, dadurch eine Kombination herstellend;
(b) Erhalten der für das Auftreten der Hybridisierung günstigen Kombinationsbedingungen; und
(c) Bestimmen, ob Hybridisierung auftritt, wobei das Auftreten von Hybridisierung anzeigt, dass das Individuum dem Risiko für die Entwicklung oder das Fortschreiten altersbedingter Makuladegeneration unterliegt; oder
(III)
(a) Sequenzieren einer Region der DNA eines Individuums, die das Nukleotid an Position 29 in SEQ ID No: 69 umfasst von dem Transkriptionsstartpunkt des Promotors des HTRA1-Gens beim Menschen; und
(b) Bestimmen, ob eine Variante der Wildtyp-Sequence der Promotorregion des HTRA1-Gens in der DNA vorliegt, wobei das Vorliegen einer Variante anzeigt, dass das Individuum dem Risiko für die Entwicklung oder das Fortschreiten einer altersbedingten Makuladegeneration unterliegt;
wobei besagte Variante eine andere Nukleotidbase ist als G an Position 29 in SEQ ID No: 69.

2. Das Verfahren nach Anspruch 1, wobei besagte Variante mit einem Single Nucleotide Polymorphism (G-→A) an Position 29 in SEQ ID No: 69 (rs11200638) korrespondiert.

3. Das Verfahren nach Anspruch 1 oder Anspruch 2, weiterhin umfassend Bestimmen des Vorliegens oder der Abwesenheit einer zusätzlichen Variante in einem Gen, das mit dem Auftreten altersbedingter Makuladegeneration bei Menschen korreliert, in der Probe, wobei die zusätzliche Variante eine andere ist als die Variante in der nicht-kodierenden regulatorischen Region strangaufwärts der Position +1 des putativen Transkriptionspunkts eines humanen HTRA1-Gens.

4. Das Verfahren nach Anspruch 3, wobei die zusätzliche Variante durch den Einsatz einer zweiten Sonde detektiert wird, die mit der zusätzlichen Variante hybridisiert.

5. Das Verfahren nach Anspruch 3, wobei die zusätzliche Variante durch den Einsatz eines zweiten Satzes von Primern, der mit einer der Seiten der zusätzlichen Variante hybridisiert, detektiert wird.

6. Das Verfahren nach einem der Ansprüche 3 bis 5, wobei die zusätzliche Variante das Vorliegen von Histidin an Position 402 des humanen CFH-Proteins ist, korrespondierend mit dem SNP rs1061170 und/oder das Vorliegen einer anderen Aminosäure als Alanin an Position 69 des humanen Proteins LOC387715, zum Beispiel Serin, korrespondierend mit SNP rs10490924.

7. Das Verfahren nach einem der Ansprüche 1 bis 6, wobei das Individuum ein asiatisches Individuum ist.

8. Ein diagnostisches Kit zum Detektieren eines abweichenden HTRA1-Gens in einer Probe eines Individuums, umfassend wenigstens ein Aufnahmemittel und einem Label und/oder Gebrauchsanweisung für das diagnostische Kit zur Detektierung eines abweichenden HTRA1-Gens in einer Probe, wobei besagtes Aufnahmemittel bereithält:
(a) eine Polynukleotidsonde, die unter strengen Bedingungen mit einer ersten Variante in der nicht-kodierenden regulatorischen Region strangaufwärts der Position +1 des putativen Transkriptionspunkts des humanen HTRA1-Gens, das mit dem Auftreten altersbedingter Makuladegeneration bei Menschen korreliert, hybridisiert; oder
(b) einen Polynukleotidprimer, der unter strengen Bedingungen angrenzend an eine Seite der Variante an Position 29 in SEQ ID No: 69 der Promotorregion des humanen HTRA1-Gens, das mit dem Auftreten altersbedingter Makuladegeneration bei Menschen korreliert, hybridisiert, und optional einen zweiten Polynukleotidprimer, der unter strengen Bedingungen mit der anderen Seite der Variante an Position 29 in SEQ ID No: 69 hybridisiert;
wobei das Kit weiterhin eine zweite Sonde oder Paar von Primern umfasst, die in der Probe das Vorliegen oder die Abwesenheit einer zusätzlichen Variante, die mit dem Auftreten altersbedingter Makuladegeneration bei Menschen korreliert, detektieren und wobei besagte erste Variante eine andere Nukleotidbase ist als G an Position 29 in SEQ ID No: 69 und besagte zusätzliche Variante das Vorliegen von Histidin an Position 402 des humanen CFH-Proteins ist, korrespondierend mit dem SNP rs1061170 und/oder das Vorliegen einer anderen Aminosäure als Alanin an Position 69 des humanen Proteins LOC387715, zum Beispiel Serin, korrespondierend mit SNP rs 10490924.

9. Eine Zusammensetzung zum Gebrauch in einem Verfahren zur Behandlung altersbedingter Makuladegeneration, umfassend einen pharmazeutisch zulässigen Träger und einen bispezifischen, einkettigen, chimären, humanisierten oder humanen Antikörper, der an das HTRA1-Polypeptid bindet oder ein Antikörper, der spezifisch reaktiv ist mit dem HTRA1-Polypeptid, wobei das Binden des Antikörpers an das HTRA1-Polypeptid die Aktivität des HTRA1-Polypeptids reduziert.

## Revendications

1. Procédé d'identification ou d'aide à l'identification d'un individu souffrant ou présentant un risque de développer une dégénérescence maculaire liée à l'âge, de diagnostic ou d'aide au diagnostic de la dégénérescence maculaire liée à l'âge chez un individu, ou d'évaluation du risque de progression de la dégénérescence maculaire liée à l'âge chez un individu, comprenant :
(I) l'analyse d'un échantillon obtenu de l'individu afin de vérifier la présence d'une base nucléotidique différente d'un G en position 29 dans la SEQ. ID N°69 du gène HTRA1 humain, dans laquelle la présence d'une base nucléotidique différente d'un G dans ladite position de la variante de gène HTRA1 indique que l'individu souffre ou présente un risque de développement ou de progression de la dégénérescence maculaire liée à l'âge ; ou
(II)
(a) la combinaison d'un échantillon provenant de l'individu avec une sonde polynucléotidique qui s'hybride, dans des conditions stringentes, à une variation dans la zone régulatrice non-codante en amont de la position +1 du site de départ de transcription putatif du gène HTRA1 humain, qui est corrélée à la survenue de la dégénérescence maculaire liée à l'âge chez les humains, mais ne s'hybride pas à un gène HTRA1 de type sauvage, produisant ainsi une combinaison ;
(b) le maintien des conditions de combinaison appropriées pour que l'hybridation ait lieu ; et
(c) la détermination du fait que l'hybridation survient ou non, dans laquelle la survenue de l'hybridation indique que l'individu présente un risque de développement ou de progression de la dégénérescence maculaire liée à l'âge ; ou
(III)
(a) le séquençage d'une zone de l'ADN provenant d'un individu qui comprend le nucléotide en position 29 dans la SEQ. ID N°69 depuis le site de départ de transcription putative du promoteur du gène HTRA1 chez les humains ; et
(b) la détermination du fait qu'une variation de la séquence de type sauvage de la zone du promoteur du gène HTRA1 est présente dans l'ADN, dans laquelle la présence de la variation indique que l'individu présente un risque de développement ou de progression de la dégénérescence maculaire liée à l'âge ;
ladite variation étant une base nucléotidique différente d'un G en position 29 dans la SEQ. ID N°69.

2. Procédé selon la revendication 1, dans lequel ladite variante correspond à un polymorphisme de nucléotide unique (G → A) en position 29 dans la SEQ. ID N°69 (rs11200638).

3. Procédé selon la revendication 1 ou la revendication 2, comprenant en outre la détermination dans l'échantillon de la présence ou de l'absence d'une variation additionnelle dans un gène, qui est corrélée à la survenue de la dégénérescence maculaire liée à l'âge chez les humains, dans lequel la variation additionnelle est différente de la variation dans la zone régulatrice non-codante en amont de la position +1 du site de transcription putatif d'un gène HTRA1 humain.

4. Procédé selon la revendication 3, dans lequel la variation additionnelle est détectée en utilisant une seconde sonde qui s'hybride à la variation additionnelle.

5. Procédé selon la revendication 3, dans lequel la variation additionnelle est détectée en utilisant un second ensemble d'amorces qui s'hybrident de chaque côté de la variation additionnelle.

6. Procédé selon l'une quelconque des revendications 3 à 5, dans lequel la variation additionnelle est la présence d'histidine en position 402 de la protéine CFH humaine, correspondant au SNP rs1061170 et/ou la présence d'un acide aminé autre que l'alanine en position 69 de la protéine humaine LOC387715, par exemple la sérine, correspondant au SNP rs10490924.

7. Procédé selon l'une quelconque des revendications 1 à 6, dans lequel l'individu est un individu asiatique.

8. Kit de diagnostic permettant de détecter une variation de gène HTRA1 dans un échantillon provenant d'un individu, comprenant au moins un système de contenant et une étiquette et/ou des instructions pour l'utilisation du kit de diagnostic dans la détection d'une variante de gène HTRA1 dans un échantillon, dans lequel ledit système de contenant dispose à l'intérieur :
(a) d'une sonde polynucléotidique qui s'hybride, dans des conditions stringentes, à une première variation dans la zone régulatrice non-codante en amont de la position +1 du site de transcription putatif du gène HTRA1 humain, qui est corrélée à la survenue de la dégénérescence maculaire liée à l'âge chez les humains ; ou
(b) d'une amorce polynucléotidique qui s'hybride, dans des conditions stringentes, de manière adjacente à un côté d'une variation en position 29 dans la SEQ. ID N°69 de la région de promotion du gène HTRA1 humain, qui est corrélée à la survenue de la dégénérescence maculaire liée à l'âge chez les humains et éventuellement d'une seconde amorce polynucléotidique qui s'hybride dans des conditions stringentes, à l'autre côté de la variation dans la position 29 de la SEQ. ID N°69 ;
dans lequel le kit comprend en outre une seconde sonde ou paire d'amorces qui détectent, dans l'échantillon, la présence ou l'absence d'une variation additionnelle qui est corrélée à la survenue de la dégénérescence maculaire liée à l'âge chez les humains et dans lequel ladite première variation est une base nucléotidique différente d'un G, en position 29 dans la SEQ. ID N°69 et ladite variation additionnelle est la présence d'histidine en position 402 de la protéine CFH humaine, correspondant au SNP rs 1061170 et/ou la présence d'un acide aminé autre que l'alanine en position 69 de la protéine humaine LOC387715, par exemple la sérine, correspondant au SNP rs10490924.

9. Composition destinée à être utilisée dans un procédé de traitement de la dégénérescence maculaire liée à l'âge, comprenant un excipient pharmaceutiquement acceptable et un anticorps humain ou humanisé, chimérique, à chaîne unique, bispécifique qui se lie au polypeptide HTRA1 ou un anticorps spécifiquement réactif avec le polypeptide HTRA1, dans laquelle la liaison de l'anticorps au polypeptide HTRA1 réduit l'activité dudit polypeptide HTRA1.
